# EUROPEAN PATENT APPLICATION

(11) **EP 4 588 939 A1**
(43) Date of publication of application: **23.07.2025**
(21) Application number: 24382041.2
(22) Date of filing: 16.01.2024
(51) Int. Cl.: C07K 16/28, A61P 35/00

(54) **INTERLEUKIN-13 RECEPTOR SUBUNIT ALPHA-2 (IL13RALPHA2) TARGETING MOIETIES FOR THE TREATMENT AND PREVENTION OF IL13RALPHA2-POSITIVE CANCER**

(71) Applicant: Universitat de Barcelona, 08028 Barcelona (ES); Fundació de Recerca Clínic Barcelona-Institut d'Investigacions Biomèdiques August Pi I Sunyer, 08036 Barcelona (ES); Hospital Clínic de Barcelona, 08036 Barcelona (ES)
(72) Inventor: ORTIZ DE LANDAZURI PASCAL, Iñaki, 08036 Barcelona (ES); JUAN OTERO, Manuel, 08036 Barcelona (ES); URIBE HERRANZ, Mireia, 08036 Barcelona (ES); CASANOVAS ALBERTÍ, Berta, 08036 Barcelona (ES); VAZQUEZ PORTERO, Mario, 08036 Barcelona (ES); ENGEL ROCAMORA, Pablo, 08036 Barcelona (ES)
(74) Representative: Hoffmann Eitle

(57) **Abstract**

The present invention provides Interleukin-13 Receptor Subunit Alpha-2 (IL13Rα2) targeting moieties, and CAR comprising them, which may be transduced or transformed into T cells. The resultant CARTs are suitable for the treatment and prevention of IL13Rα2-positive cancers, such as glioblastoma or diffuse intrinsic pontine glioma.

## Description

### TECHNICAL FIELD

The present invention relates to the field of cancer immunotherapy. Particularly, the present invention relates to IL13Rα2 targeting moieties and their use in the treatment and prevention of IL13Rα2-positive cancers.

### BACKGROUND ART

Malignant glioma is one of the most aggressive human tumors that arises from the neuroectoderm. The multiformity and variability of these glioma tumors are present at the histomorphological and genetic molecular level; moreover, the heterogeneous morphology is also exhibited both between different patients and between different tumor areas. The most outstanding characteristic of malignant glioma is its infiltrative and invasive nature, leading to a high recurrence rate and a dismal prognosis. From them, glioblastoma multiforme (GBM), also referred to as a grade IV astrocytoma, is a fast-growing and aggressive brain tumor.

As initial treatment approach, GBM is treated with surgery. However, surgery does not lead to the curation of the disease. Radiotherapy is another conventional approach after surgery, although radio-chemotherapy appears to be more effective than radiation therapy alone. Similarly, chemotherapy is an alternate conventional therapy choice, but this approach is less effective for GBM therapy because of the short plasma half-lives of chemotherapy drugs, poor solubility and permeability across the blood-brain barrier and blood-brain-tumor barrier. Due to low efficacy of conventional treatment, new therapies and genetic treatments are urgently needed for patients with GBM.

Diffuse intrinsic pontine gliomas (DIPGs) are highly-aggressive and difficult-to-treat brain tumors found at the base of the brain. They are also glial tumours, and their current treatment is based in surgery, radiation therapy or chemotherapy. However, complete surgical removal is not possible due to their location in the brain stem, and the radiation therapy's response is short-lived, lasting about six to nine months on average. Similarly as for GBM, there is a urgent need for new therapeutic approaches for patients with DIPGs.

Interleukin-13 receptor subunit alpha-2 (IL13Rα2), an immunotherapeutic target that is the high affinity receptor for interleukin-13, is considered a promising direction for the treatment of gliomas. IL13Rα2 is abundantly expressed in a variety of tumors, such as pancreatic cancer, ovarian cancer, breast cancer, colon cancer and melanoma. Recently, several researchers found that IL13Rα2 could serve as a brand-new target in immunotherapy trials of malignant gliomas. This is because it is believed that IL13Rα2 is highly expressed in these tumors and it is related to poor survival, promoting glioma proliferation and invasion. In contrast, normal tissues express little to no IL13Rα2.

One approach for targeting this tumor-specific receptor utilizes the cognate ligand, IL-13, conjugated to cytotoxic molecules. This approach, however, lacks specificity because the lower affinity receptor for IL-13, IL13Rα1, is widely expressed by normal tissues. Some mutations aimed at increasing the affinity of IL13 for IL13Rα2 have been described.

Previously, CAR immunotherapy has shown effective outcomes in the treatment and management of tumors based on lymphocytic recognition of cancers cells and its consecutive destruction by immune system. However, the use of CAR therapy beyond hematologic cancers and the efficacy of this therapy against solid tumors remain to be established. Thus, further studies of CAR immunotherapy in solid tumours such as GBM and DIPG are needed, and IL13Rα2 is a promising candidate, since it is expressed at a high frequency in these aggressive and incurable forms of primary brain tumors, as well as by other solid tumors.

### SUMMARY OF THE INVENTION

The present invention provides an **IL13Rα2 targeting moiety** that is an antibody, F(ab')2, Fab, preferably scFv, or scFab comprising:
- a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 1, LCDR2 consists of SEQ ID NO: 2, LCDR3 consists of SEQ ID NO: 3, HCDR1 consists of SEQ ID NO: 4, HCDR2 consists of SEQ ID NO: 5, and HCDR3 consists of SEQ ID NO: 6; or
- a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 17, LCDR2 consists of SEQ ID NO: 18, LCDR3 consists of SEQ ID NO: 19, HCDR1 consists of SEQ ID NO: 20, HCDR2 consists of SEQ ID NO: 21, and HCDR3 consists of SEQ ID NO: 22; or
- a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 33, LCDR2 consists of SEQ ID NO: 34, LCDR3 consists of SEQ ID NO: 35, HCDR1 consists of SEQ ID NO: 36, HCDR2 consists of SEQ ID NO: 37, and HCDR3 consists of SEQ ID NO: 38; or
- a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 49, LCDR2 consists of SEQ ID NO: 50, LCDR3 consists of SEQ ID NO: 51, HCDR1 consists of SEQ ID NO: 52, HCDR2 consists of SEQ ID NO: 53, and HCDR3 consists of SEQ ID NO: 54.

Preferably, the IL13Rα2 targeting-moiety is an scFv comprising a VL and VH domains, and wherein:
- the VL domain comprises a sequence with at least 85% sequence identity to SEQ ID NO: 7, and wherein said VL domain comprises CDR polypeptides of LCDR1 according to SEQ ID NO: 1, LCDR2 according to SEQ ID NO: 2, and LCDR3 according to SEQ ID NO: 3, and said VH domain comprises a sequence with at least 80% sequence identity to SEQ ID NO: 8, and wherein said VH domain comprises CDR polypeptides of HCDR1 according to SEQ ID NO: 4, HCDR2 according to SEQ ID NO: 5, and HCDR3 according to SEQ ID NO: 6; or
- the VL domain comprises a sequence with at least 85% sequence identity to SEQ ID NO: 23, and wherein said VL domain comprises CDR polypeptides of LCDR1 according to SEQ ID NO: 17, LCDR2 according to SEQ ID NO: 18, and LCDR3 according to SEQ ID NO: 19, and said VH domain comprises a sequence with at least 80% sequence identity to SEQ ID NO: 24, and wherein said VH domain comprises CDR polypeptides of HCDR1 according to SEQ ID NO: 20, HCDR2 according to SEQ ID NO: 21, and HCDR3 according to SEQ ID NO: 22; or
- the VL domain comprises a sequence with at least 85% sequence identity to SEQ ID NO: 39, and wherein said VL domain comprises CDR polypeptides of LCDR1 according to SEQ ID NO: 33, LCDR2 according to SEQ ID NO: 34, and LCDR3 according to SEQ ID NO: 35, and said VH domain comprises a sequence with at least 80% sequence identity to SEQ ID NO: 40, and wherein said VH domain comprises CDR polypeptides of HCDR1 according to SEQ ID NO: 36, HCDR2 according to SEQ ID NO: 37, and HCDR3 according to SEQ ID NO: 38; or
- the VL domain comprises a sequence with at least 85% sequence identity to SEQ ID NO: 55 and wherein said VL domain comprises CDR polypeptides of LCDR1 according to SEQ ID NO: 49, LCDR2 according to SEQ ID NO: 50, and LCDR3 according to SEQ ID NO: 51, and said VH domain comprises a sequence with at least 80% sequence identity to SEQ ID NO: 56, and wherein said VH domain comprises CDR polypeptides of HCDR1 according to SEQ ID NO: 52, HCDR2 according to SEQ ID NO: 53, and HCDR3 according to SEQ ID NO: 54.

Preferably, the IL13Rα2 targeting moiety is a scFv comprising a VL domain and VH domain, wherein:
- the VL domain consists of SEQ ID NO: 7 and the VH domain consists of SEQ ID NO: 8; or
- the VL domain consists of SEQ ID NO: 9 and the VH domain consists of SEQ ID NO: 10; or
- the VL domain consists of SEQ ID NO: 23 and the VH domain consists of SEQ ID NO: 24; or
- the VL domain consists of SEQ ID NO: 25 and the VH domain consists of SEQ ID NO: 26; or
- the VL domain consists of SEQ ID NO: 39 and the VH domain consists of SEQ ID NO: 40; or
- the VL domain consists of SEQ ID NO: 41 and the VH domain consists of SEQ ID NO: 42; or
- the VL domain consists of SEQ ID NO: 55 and the VH domain consists of SEQ ID NO: 56; or
- the VL domain consists of SEQ ID NO: 57 and the VH domain consists of SEQ ID NO: 58.

Preferably, the IL13Rα2 targeting-moiety is a scFv consisting of SEQ ID NO: 13, SEQ ID NO: 15, SEQ ID NO: 29, SEQ ID NO: 31, SEQ ID NO: 45, SEQ ID NO: 47, SEQ ID NO: 61, or SEQ ID NO: 63.

The present invention also provides a chimeric antigen receptor (CAR) comprising:
a) an extracellular domain comprising a IL13Rα2 targeting moiety as defined in herein;
b) a transmembrane domain; and
c) an intracellular signaling domain.

Preferably, the transmembrane domain comprises the transmembrane domain of CD8, preferably the transmembrane domain of CD8 comprising SEQ ID NO: 67. Preferably, the intracellular signaling domain comprises the intracellular domain of CD3ζ, preferably the intracellular domain of CD3ζ comprising SEQ ID NO: 68. Preferably, the CAR further comprises a costimulatory signaling domain, preferably the costimulatory signaling domain comprises the intracellular domain of CD137, preferably the intracellular domain of CD137comprising SEQ ID NO: 69.

Preferably, the CAR comprises or consists of SEQ ID NO: 14, SEQ ID NO: 16, SEQ ID NO: 30, SEQ ID NO: 32, SEQ ID NO: 46, SEQ ID NO: 48, SEQ ID NO: 62, or SEQ ID NO: 64.

The present invention also provides a nucleic acid encoding the CAR defined herein.

The present invention also provides a cell comprising the nucleic acid according to the second aspect and/or the CAR defined above. Preferably, the cell is a T cell.

The present invention also provides a pharmaceutical composition comprising a plurality of cells as defined herein.

The present invention also provides the IL13Rα2 targeting moiety, the CAR, the nucleic acid, the cell, or the pharmaceutical composition as defined herein, for use in therapy or as a medicament. Preferably, the use is in a method of treating or preventing a IL13Rα2-positive cancer, wherein the method comprises administering the IL13Rα2 targeting moiety, the CAR, the nucleic acid, the cell or the composition to a patient in need thereof. More preferably, the IL13Rα2-positive cancer is glioblastoma or diffuse intrinsic pontine glioma.

### BRIEF DESCRIPTION OF DRAWINGS

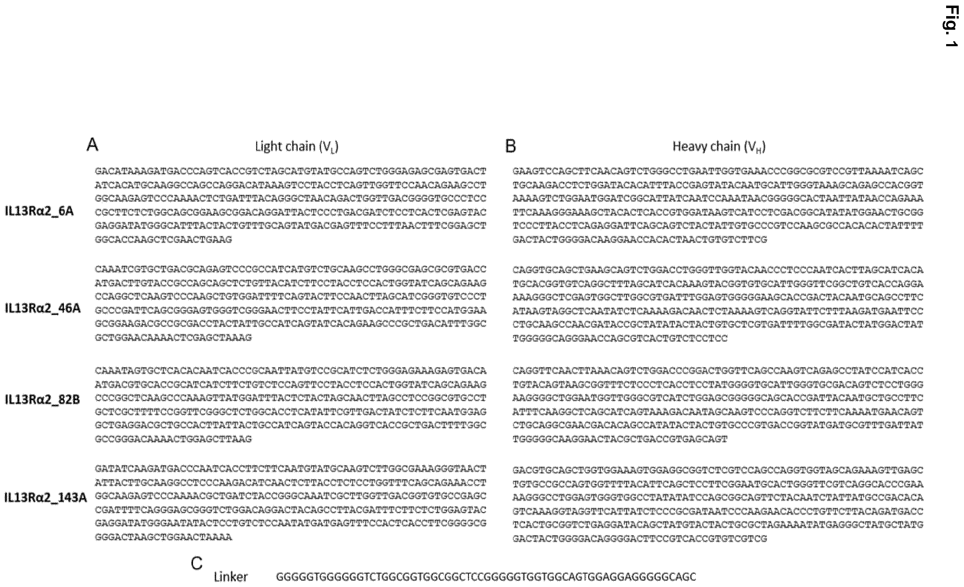

**Fig. 1****: Nucleotide sequence of the generated anti-IL13Rα2 scFvs.** Nucleotide sequence of A) light chain (VL) and B) heavy chain (VH) variable regions from IL13Rα2_6A, IL13Rα2_46A, IL13Rα2_82B and IL13Rα2_143A hybridomas. C) Nucleotide linker sequence used between light and heavy chain variable regions.

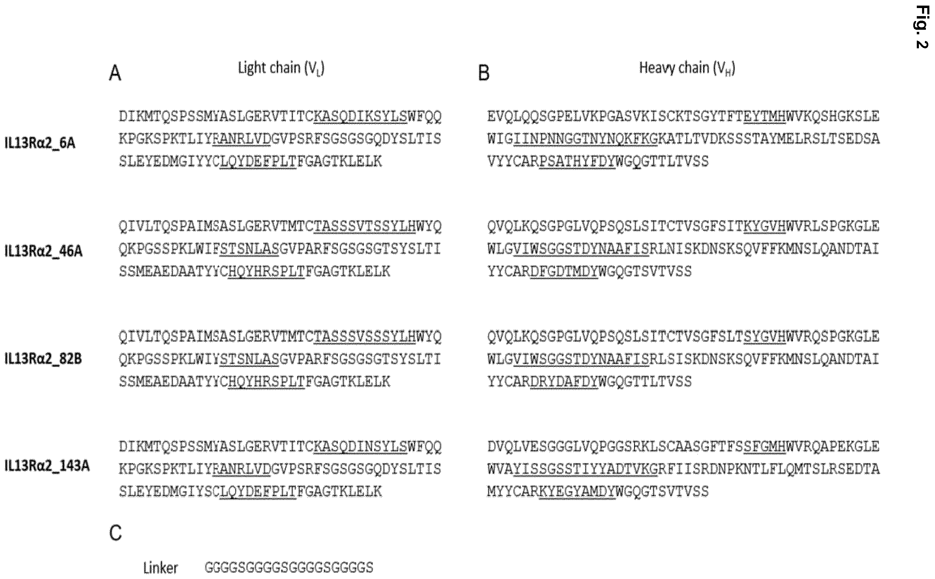

**Fig. 2****: Amino acid sequence of the generated anti-IL13Rα2 scFvs.** Amino acid sequence of A) light chain (VL) and B) heavy chain (VH) variable regions from IL13Rα2_6A, IL13Rα2_46A, IL13Rα2_82B and IL13Rα2_143A hybridomas. Underlined shows CDRs. C) Amino acid sequence of the linker sequence used between light and heavy chain variable regions.

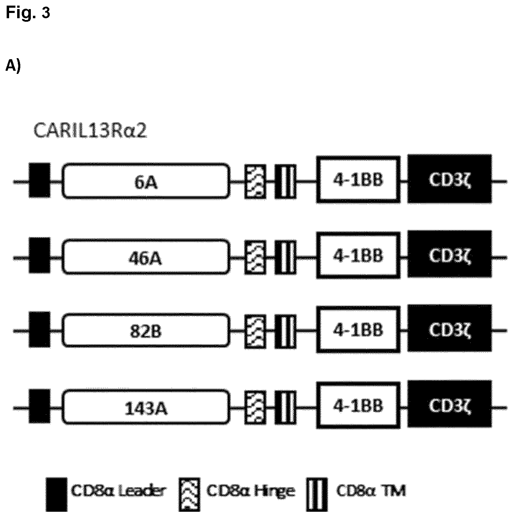

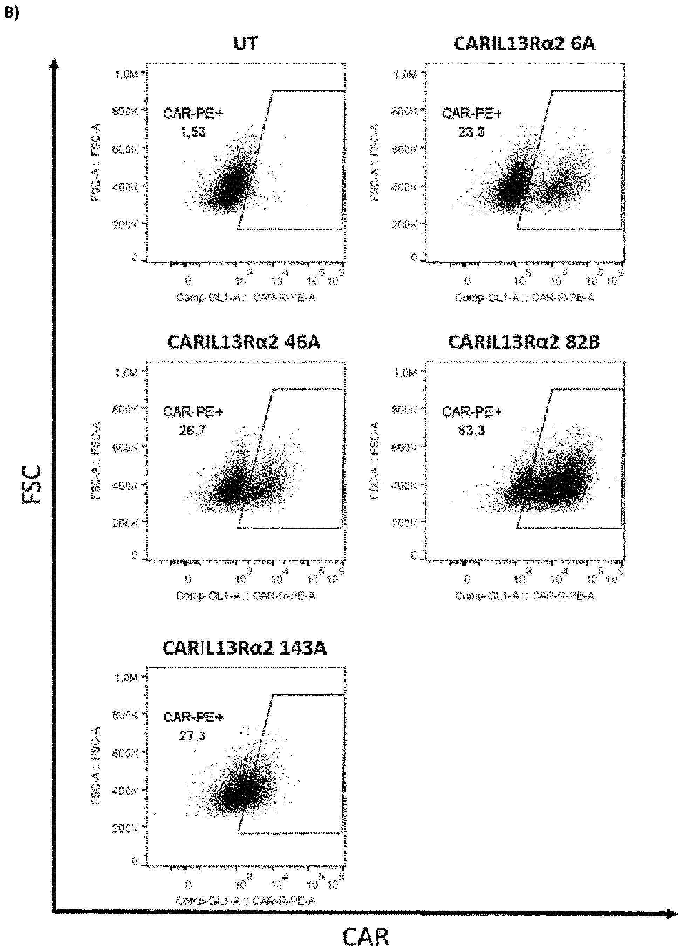

Fig. **3****: Characterization of T cells expressing the CARIL13Rα2 constructs.** A) Schematic diagram of second-generation CARs designed for this study. Each CAR differs in the scFv: 6A, 46A, 82B, and 143A. All CARs contain the CD8 hinge and transmembrane domain (TM), and the 4-1BB and CD3ζ intracellular domains. B) Representative dot plots showing expression of CAR (%) on the T cell surface assessed by flow cytometry at day 8 of an expansion culture. UT: Untransduced T cell control.

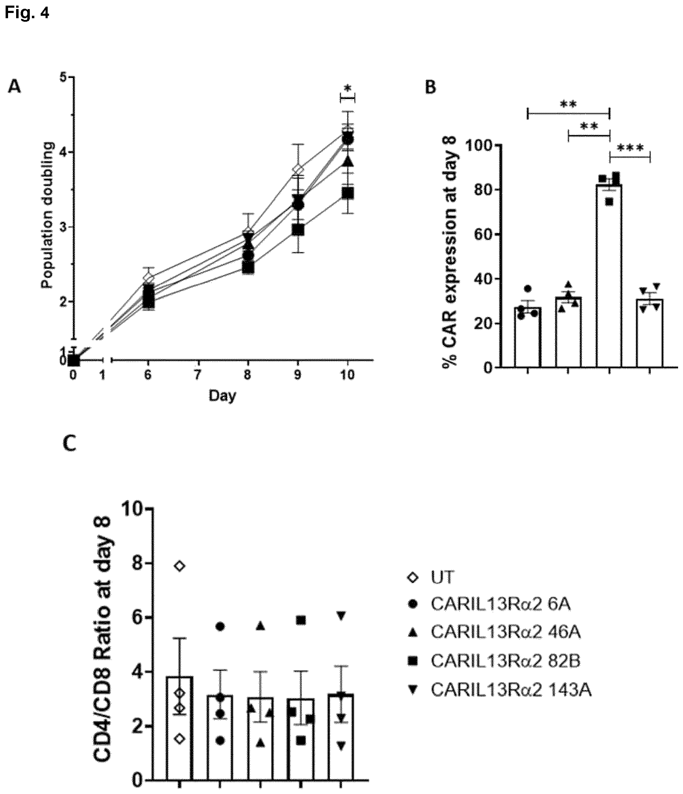

**Fig. 4****: *In vitro* characterization of CARIL13Rα2 T cells.** A) Population doublings of CARIL13Rα2 T cells at different timepoints during ex vivo expansion after stimulation with anti-CD3/CD28 beads and IL-2. At day 10, CARIL13Rα2_143A showed a higher population doubling level compared to CARIL13Rα2_82B. All data are means ± SEM from N=4. RM two-way ANOVA with the Geisser-Greenhouse correction. Tukey's multiple comparison test, with individual variances computed for each comparison. B) Quantification of the CAR expression on the T cell surface and the C) CD4/CD8 ratio assessed by flow cytometry at day 8. Each symbol represents a different normal donor. UT: Untransduced T cell control. N=4; Mean ± SEM. One-way ANOVA. * indicates p<0.05; ** indicates p<0.01; *** indicates p<0.001.

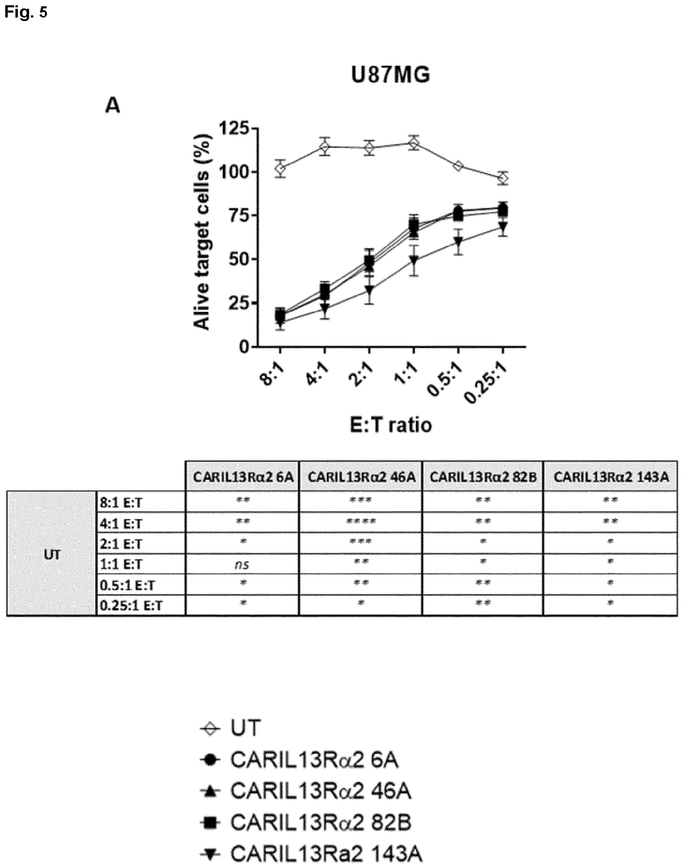

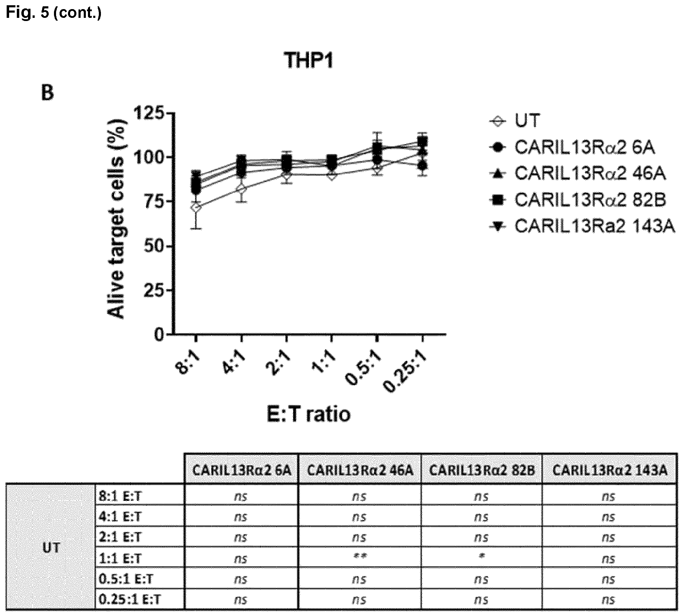

**Fig. 5****: Functional characterization of CARIL13Rα2 T cell anti-tumor activity *in vitro.*** A) CARIL13Rα2 T cells specifically kill IL13Rα2+ target cells (U87MG cell line), B) while IL13Rα1+/IL13Rα2- target cells (THP1 cell line) remain almost intact. Indicated CARIL13Rα2 T cells and target tumor cells were mixed at a fixed effector: target ratio (E:T) of 8:1, 4:1, 2:1, 1:1, 0.5:1 and 0.25:1. Cytotoxicity was evaluated 24 hours later in a luciferase-based assay, determining the percentage of target cells alive. UT: Untransduced control T cells. Mean ± SEM of 4 experiments with different normal donors. * Indicates p< 0.05; ** indicates p<0.01; *** indicates p<0.001; **** indicates p<0.0001; ns indicates not significant. Two-way ANOVA with the Geisser-Greenhouse correction. Tukey's multiple comparison test, with individual variances computed for each comparison.

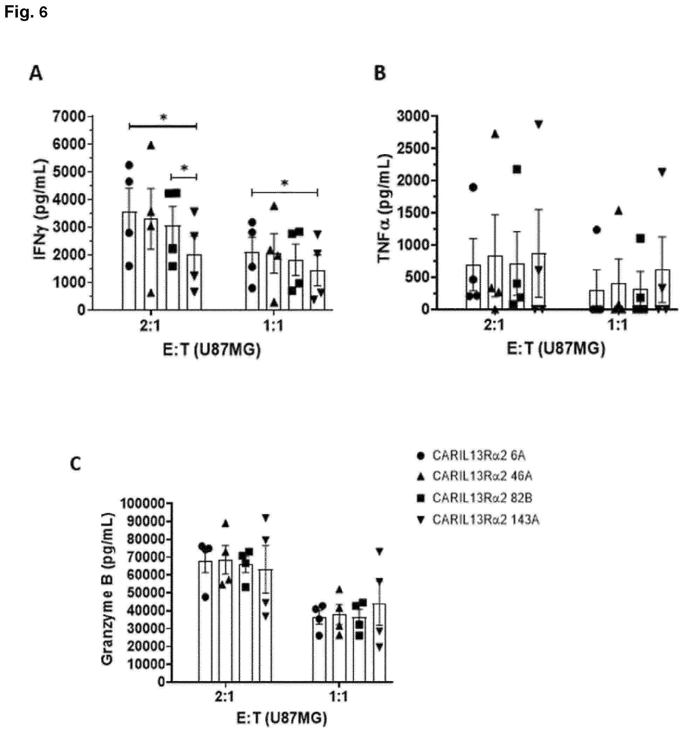

**Fig. 6****: Cytokine production of CARIL13Rα2 T cells *in vitro.*** A) IFNγ, B) TNFα and C) Granzyme B release of CARIL13Rα2 T cells after 24 hours in coculture with U87MG cells, measured by ELISA. Each symbol represents a different normal donor at a fixed effector: target ratio (E:T). Mean ± SEM of 4 experiments. * indicates p< 0.05. TNFα levels lower than the ELISA detection limit were plotted as 0 pg/mL. Two-way ANOVA with the Geisser-Greenhouse correction. Tukey's multiple comparison test, with individual variances computed for each comparison.

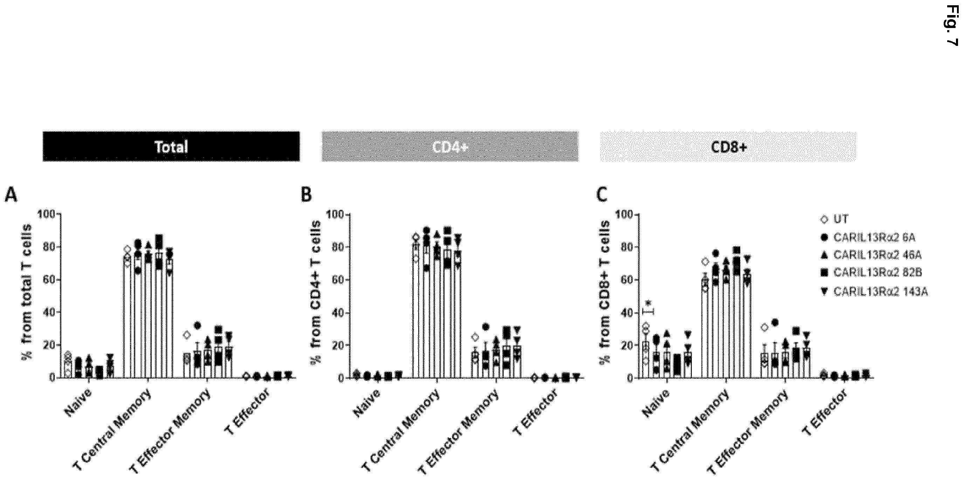

**Fig. 7****: Immunophenotypic characterization of CARIL13Rα2 T cell subsets.** Naive (CD62L+/CD45RA+), Central Memory (CD62L+/CD45RA-), Effector Memory (CD62L-/CD45RA-) and Effector (CD62L-/CD45RA+) subpopulations were reported in A) the total T cell population and in B) CD4+ or in C) CD8+ T cells after 10 days of expansion. Each symbol represents a different normal donor. UT: Untransduced control T cells. Mean ± SEM of 4 experiments. * Indicates p< 0.05. Two-way ANOVA with the Geisser-Greenhouse correction. Tukey's multiple comparison test, with individual variances computed for each comparison.

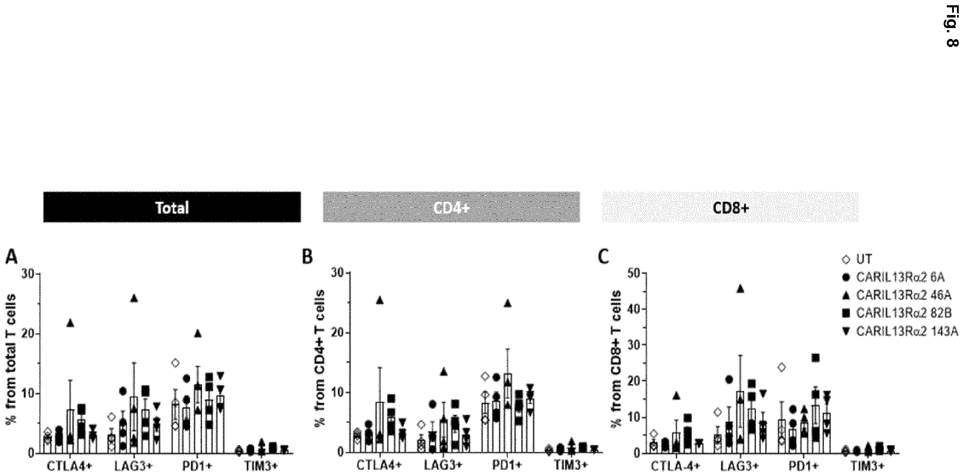

**Fig. 8****: Immunophenotypic characterization of exhaustion markers in CARIL13Rα2 T cells.** Quantification of CTLA4, LAG3, PD1 and TIM3 expression in A) the total T cell population and in B) CD4+ or in C) CD8+ T cells after 10 days of an expansion culture. Each symbol represents a different normal donor. UT: Untransduced control T cells. Mean ± SEM of 4 experiments. Two-way ANOVA with the Geisser-Greenhouse correction. Tukey's multiple comparison test, with individual variances computed for each comparison.

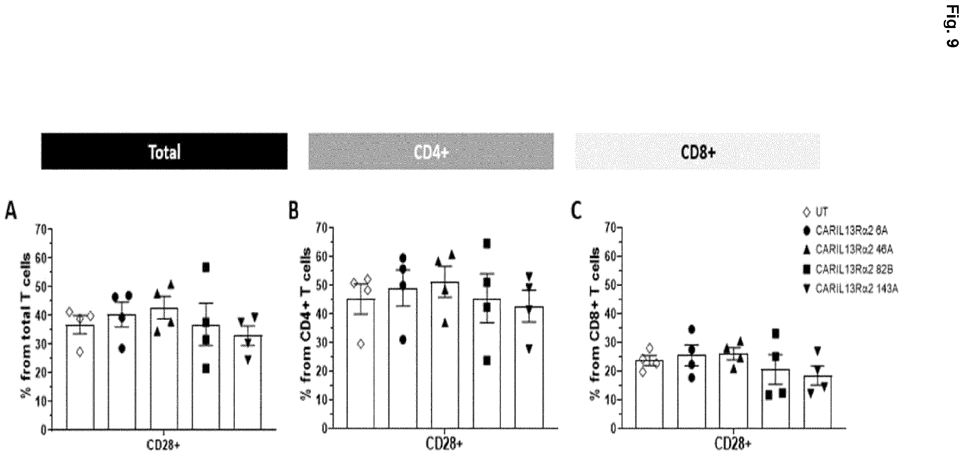

**Fig. 9****: Surface expression of CD28 marker in CARIL13Rα2 T cells.** Quantification of the CD28 surface expression in A) the total T cell population and in B) CD4+ or C) CD8+ T cells after 10 days of expansion. Each symbol represents a different normal donor. UT: Untransduced control T cells. Mean ± SEM of 4 experiments. Two-way ANOVA with the Geisser-Greenhouse correction. Tukey's multiple comparison test, with individual variances computed for each comparison.

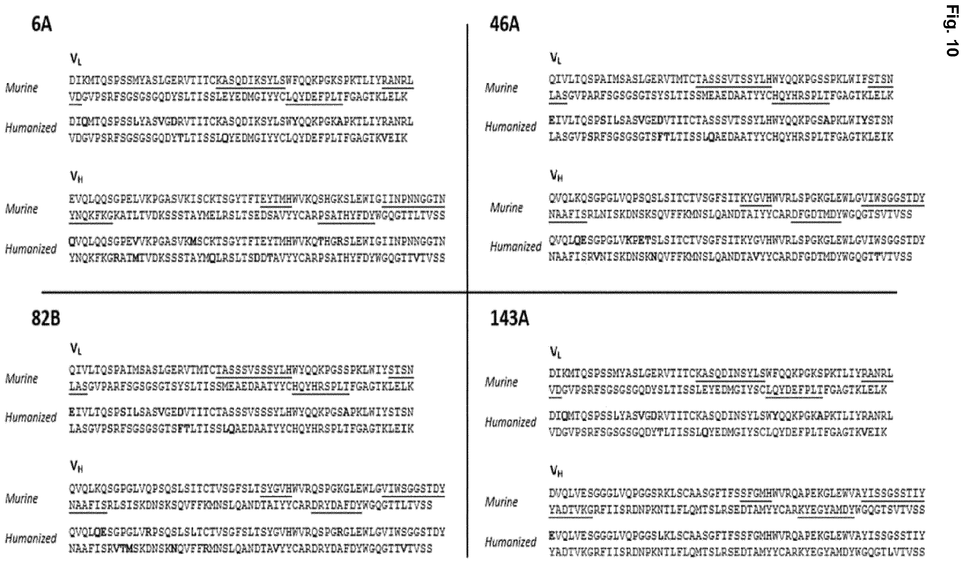

**Fig. 10****: Amino acid sequences of the designed humanized anti-IL13Rα2 scFvs.** Murine sequences were humanized by CDR grafting method, while retaining those murine framework residues that influence the antigen-binding activity and the structural configuration. A) Light chain (VL) and B) Heavy chain (VH) variable region amino acid sequence of humanized anti-IL13Rα2 scFvs: 6A, 46A, 82B and 143A. Underlined shows CDRs in the murine sequences. In bold, amino acid changes in the humanized sequences of the anti-IL13Rα2 scFv.

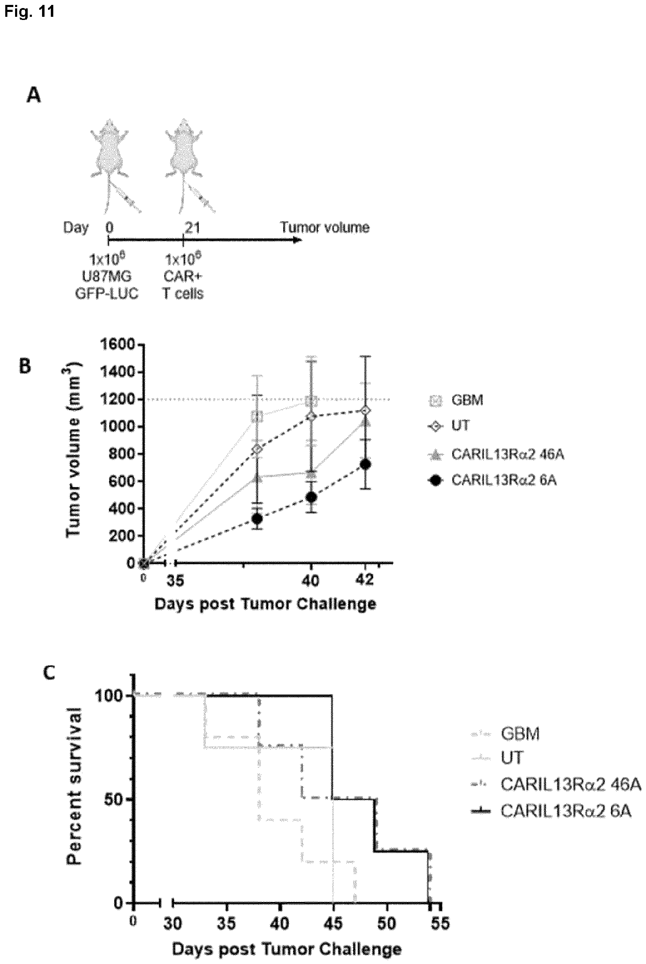

**Fig. 11****: CARIL13Rα2_6A and 46A T cells and GBM progression in a mouse xenograft model after a single intravenous injection of 1×10⁶ CAR+ T cells.** A) Schematic procedure to generate a GBM mouse xenograft model. NSG mice were injected subcutaneously with 1×10⁶ U87MG GFP-LUC cells. 21 days later, animals were treated with a single intravenous injection of 1×10⁶ of untransduced (UT) or CARIL13Rα2_6A+ or CARIL13Rα2_46A+ T cells. Tumor growth was monitored twice a week via caliper (tumor volume). B) Overall kinetics of tumor progression in mice during 4 weeks after treatment (7 weeks after Tumor challenge). All data are means ± SEM. Two-way ANOVA. C) Kaplan-Meier survival curve (log-rank test) of mice untreated (GBM) or treated with UT control or CARIL13Rα2_6A or CARIL13Rα2_46A T cells. (N=4 for each group).

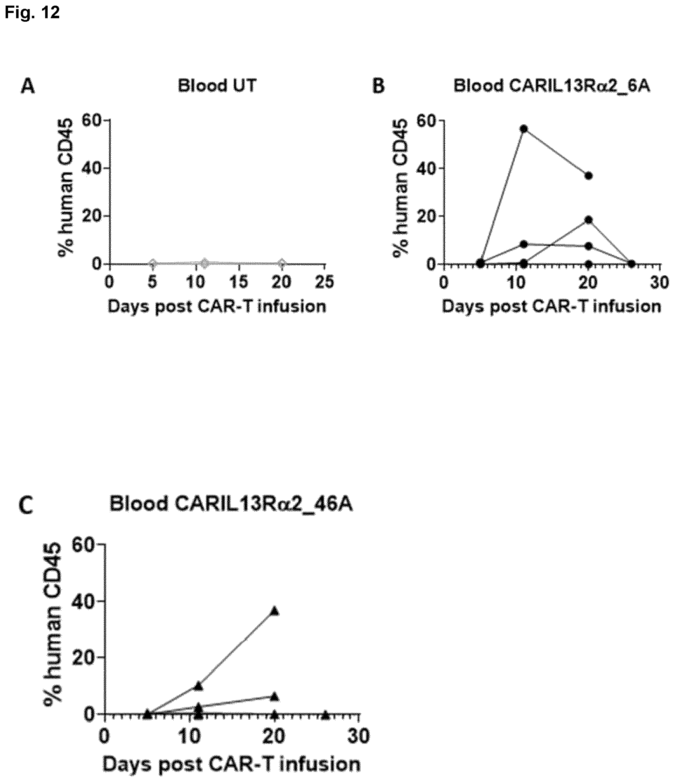

**Fig. 12****: T cell persistence after a single intravenous injection of 1×10⁶ CARIL13Rα2+ T cell treatment.** Blood was collected by submaxillary bleed on the indicated days and the persistence of A) untransduced (UT) , B) CARIL13Ra2_6A and C) CARIL13Rα2_46A T cells was analyzed. % of peripheral blood CD45+ T cell gated inside live cells are plotted. Each continuous line represents a different individual, and each symbol represents a measurement.

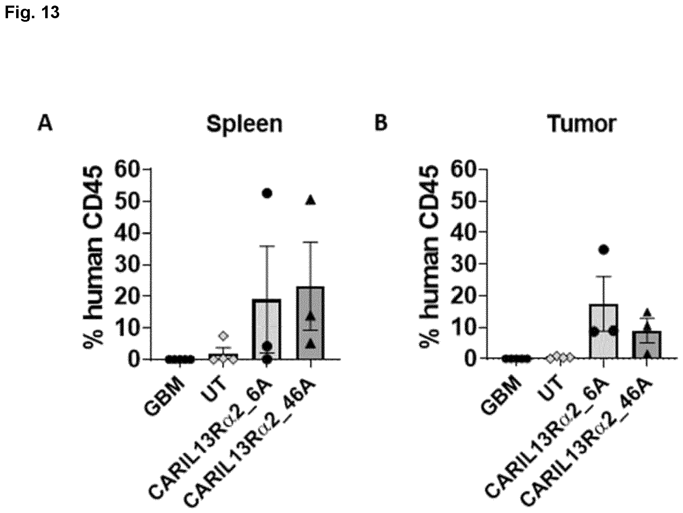

**Fig. 13****: T cell infiltration at the end point of the experiment in spleen and tumor after a single intravenous injection of 1×10⁶ CARIL13Rα2+ T cell treatment.** A) Spleen and B) tumor were collected at the end point of the experiment to assess T cell infiltration. % of CD45+ T cell gated inside live cells are plotted. GBM: untreated. UT: untransduced T cells. All data are means ± SEM. Each symbol represents a different individual. One-way ANOVA.

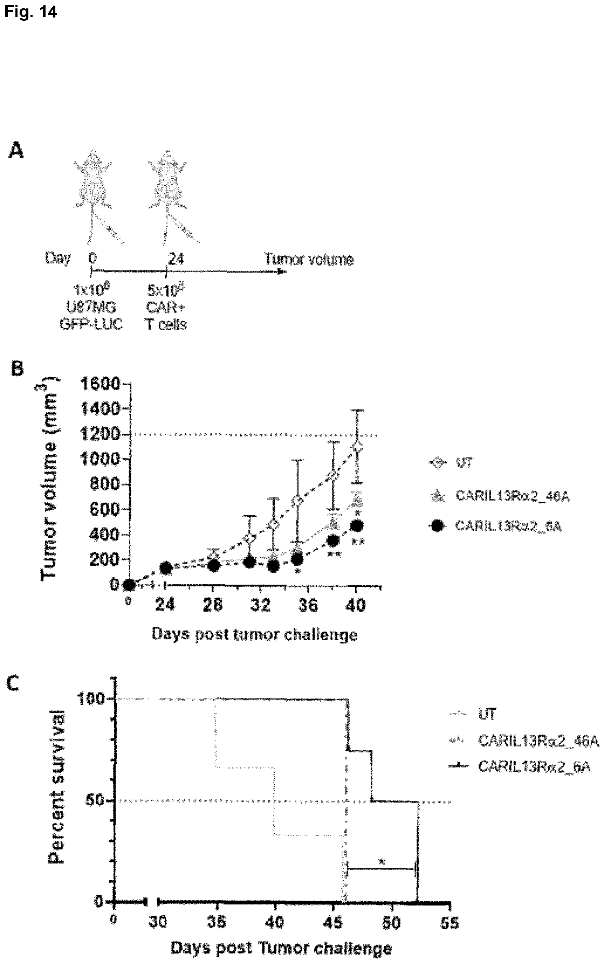

**Fig. 14****: CARIL13Rα2_6A and 46A T cells and GBM progression in a mouse xenograft model after a single intravenous injection of 5×10⁶ CAR+ T cells.** A) Schematic procedure to generate a GBM mouse xenograft model. NSG mice were injected subcutaneously with 1×10⁶ U87MG GFP-LUC cells. 24 days later, animals were treated with a single intravenous injection of 5×10⁶ of untransduced (UT) or CARIL13Rα2_6A+ or CARIL13Rα2_46A+ T cells. Tumor growth was monitored twice a week via caliper (tumor volume). B) Overall kinetics of tumor progression in mice during 3 weeks after treatment (7 weeks after Tumor challenge). All data are means ± SEM. Two-way ANOVA. C) Kaplan-Meier survival curve (log-rank test) of mice treated with UT control or CARIL13Rα2_6A or CARIL13Rα2_46A T cells. (N=3 for each group). * indicates p< 0.05; ** indicates p<0.01.

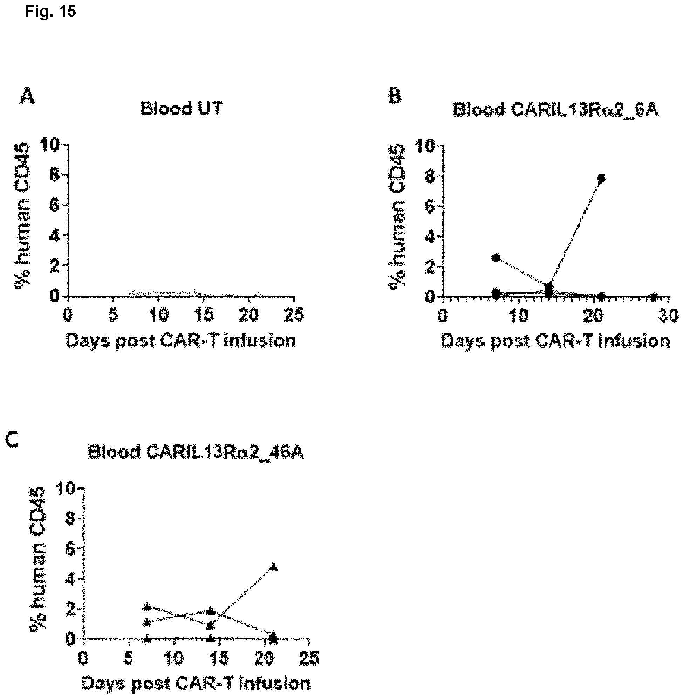

**Fig. 15****: T cell persistence after a single intravenous injection of 5×10⁶ CARIL13Rα2+ T cell treatment.** Blood was collected by submaxillary bleed on the indicated days and the persistence of A) untransduced (UT), B) CARIL13Ra2_6A and C) CARIL13Rα2_46A T cells was analyzed. % of peripheral blood CD45+ T cell gated inside live cells are plotted. Each continuous line dot represents a different individual, and each symbol represents a measurement.

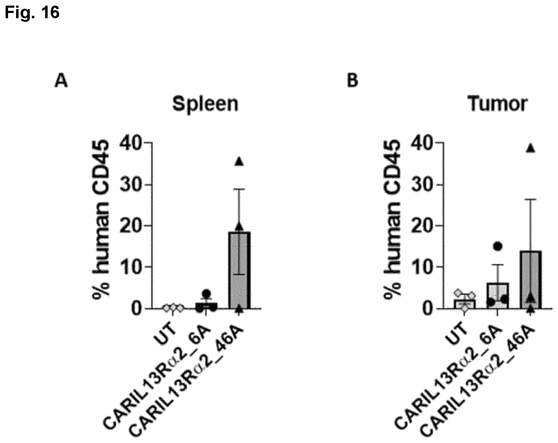

**Fig. 16****: T cell infiltration at the end point of the experiment in spleen and tumor after a single intravenous injection of 5×10⁶ CARIL13Rα2+ T cell treatment.** A) Spleen and B) tumor were collected at the end point of the experiment to assess T cell infiltration. % of CD45+ T cell gated inside live cells are plotted. UT: untransduced T cells. All data are means ± SEM. Each symbol represents a different individual. One-way ANOVA.

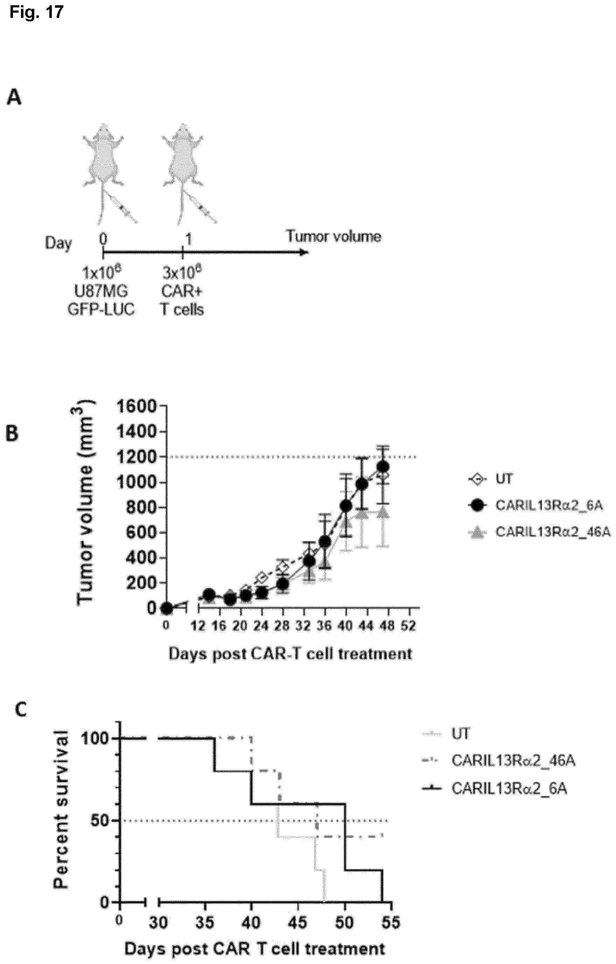

**Fig. 17****: CARIL13Rα2_6A and CARIL13Rα2_46A T cells and GBM progression in a mouse xenograft model with a co-injection of tumor cells and a single intravenous injection of 3×10⁶ CAR+ T cells.** A) Schematic procedure to generate a GBM mouse xenograft model. NSG mice were injected subcutaneously with 1×10⁶ U87MG GFP-LUC cells. 24 hours later, animals were treated with a single intravenous injection of 3×10⁶ of untransduced (UT) or CARIL13Rα2_6A+ or CARIL13Rα2_46A+ T cells. Tumor growth was monitored twice a week via calliper (tumor volume). B) Overall kinetics of tumor progression in mice during 7 weeks after treatment. All data are means ± SEM. Two-way ANOVA. C) Kaplan-Meier survival curve (log-rank test) of mice treated with UT or CARIL13Ra2_6A or CARIL13Rα2_46A T cells (N=5 for each group).

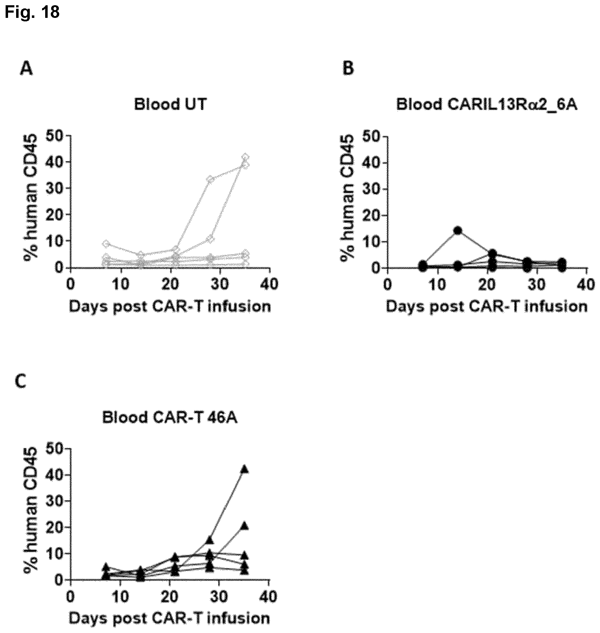

**Fig. 18****: T cell persistence after a co-injection of U87MG tumor cells and a single intravenous injection of 3×10⁶ CARIL13Rα2+ T cell treatment.** Blood was collected by submaxillary bleed on the indicated days and T cell persistence of A) untransduced (UT), B) CARIL13Ra2_6A and C) CARIL13Rα2_46A T cells was analyzed. % of peripheral blood CD45+ T cell gated inside live cells are plotted. Each continuous line dot represents a different individual, and each symbol represents a measurement.

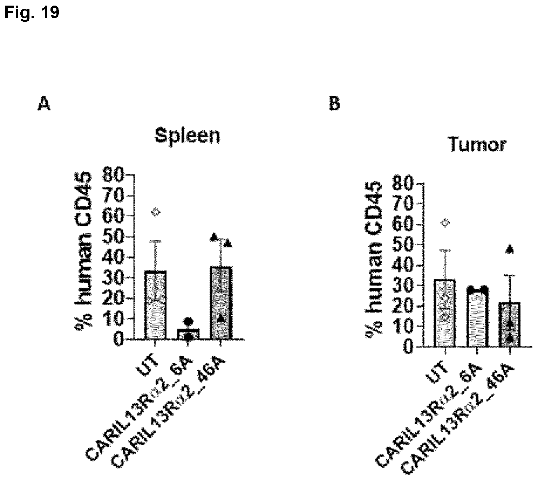

**Fig. 19****: T cell infiltration at the end point of the experiment in spleen and tumor after a co-injection of U87MG tumor cells and a single intravenous injection of 3×10⁶ CARIL13Rα2+ T cell treatment.** A) Spleen and B) tumor were collected at the end point of the experiment to assess T cell infiltration. % of CD45+ T cell gated inside live cells are plotted. UT: untransduced T cells. All data are means ± SEM. Each symbol represents a different individual. One-way ANOVA.

### GENERAL DEFINITIONS

"Administering" or "administration of" a medicament to a patient (and grammatical equivalents of this phrase) refers to direct administration, which may be administration to a patient by a medical professional, and/or indirect administration, which may be the act of prescribing a drug. E.g., a physician who instructs a patient to self-administer a medicament or provides a patient with a prescription for a drug is administering the drug to the patient.

In the context of a protein sequence or polypeptide, a "domain" refers to a functional and/or structural unit or region in a protein or polypeptide. Domains are usually responsible for a particular function or interaction, contributing to the overall role of a protein. A domain may vary in length but is preferably between 25 amino acids to up to 500 amino acids long.

The term "affibody" refers to a protein that is derived from the Z domain of protein A and that has been engineered to bind to a specific target (see Frejd & Kim, 2017. Exp Mol Med. 49(3): e306).

The term "antibody" refers to a molecule comprising at least one immunoglobulin domain that binds to, or is immunologically reactive with, a particular target. The term includes whole antibodies and any antigen binding portion or single chains thereof and combinations thereof; for instance, the term "antibody" includes bivalent antibodies and bivalent bispecific antibodies.

The term "IL13Rα2" or "interleukin-13 receptor subunit α-2" is a glioma restricted cell-surface protein that is expressed on >80% of high-grade gliomas including GBM.

IL13Rα2 is expressed by stem-like tumor cells, and in differentiated malignant cells is most closely associated with expression of mesenchymal signature genes. IL13Rα2 is composed of 3 extracellular domains (an S-type immunoglobulin fold domain called D1, and two fibronectin type-III domains, called D2 and D3), a transmembrane region, and a short cytoplasmic tail. The IL-13 binding site III, which recognizes the CD loop of IL-13, is in the D1 domain. IL-13 binding site II, which interacts with A and D helixes of IL-13, is found in the interface of D2 and D3 domains. In the cytoplasmic tail there is a phosphorylated tyrosine (Y369) required for signal transduction.

By "capable of binding to human IL13Rα2" is meant that the IL13Rα2 targeting moiety has binding affinity for human IL13Rα2. Preferably, the binding affinity is specific, i.e., the IL13Rα2 targeting moiety is capable of specifically binding to the human IL13Rα2 (which is its target molecule) in a manner which distinguishes from the binding to non-target molecules, i.e., from other molecules that are not IL13Rα2. Thus, the IL13Rα2 targeting moiety either does not bind to non-target molecules or exhibits negligible or substantially reduced (as compared to the target) e. g. background, binding to non-target molecules. The IL13Rα2 targeting moiety specifically recognises IL13Rα2. Specific binding between the IL13Rα2 targeting moiety and the human IL13Rα2 can be assessed by numerous techniques, such as surface plasmon resonance, flow cytometry, or ELISA assays.

A typical type of antibody comprises at least two heavy chains ("HC") and two light chains ("LC") interconnected by disulfide bonds.

Each "heavy chain" comprises a "heavy chain variable domain or region " (abbreviated herein as "VH") and a "heavy chain constant domain or region" (abbreviated herein as "CH"). The heavy chain constant domain typically comprises three constant domains, CH1, CH2, and CH3.

Each "light chain" comprises a "light chain variable domain or region" (abbreviated herein as "VL") and a "light chain constant domain or region" ("CL"). The light chain constant domain (CL) can be of the kappa type or of the lambda isotype. The VH and VL domains can be further subdivided into regions of hypervariability, termed Complementarity Determining Regions ("CDR"), interspersed with regions that are more conserved, termed "framework regions" ("FW"). In molecular biology, a framework region is a subdivision of the variable region (Fab) of the antibody. The variable region is composed of seven regions, four of which are framework regions and three of which are hypervariable regions. The framework region makes up about 85% of the variable region and are responsible for acting as a scaffold for the CDR polypeptides. These CDRs are in direct contact with the antigen and are involved in binding antigen, while the framework regions support the binding of the CDR to the antigen and may aid in maintaining the overall structure of the four variable domains on the antibody.

Each VH and VL is composed of three CDRs and four FWs, arranged from amino-terminus to carboxy-terminus in the following order: FW1, CDR1, FW2, CDR2, FW3, CDR3, FW4. The present disclosure inter alia presents VH and VL sequences as well as the subsequences corresponding to CDR1, CDR2, and CDR3.

The precise amino acid sequence boundaries of a given CDR can be determined using any of several well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme). Preferably, the numbering scheme used herein is Kabat.

Accordingly, a person skilled in the art would understand that the sequences of FW1, FW2, FW3 and FW4 are equally disclosed. For a particular VH, FW1 is the subsequence between the N-terminus of the VH and the N-terminus of H-CDR1, FW2 is the subsequence between the C-terminus of H-CDR1 and the N-terminus of H-CDR2, FW3 is the subsequence between the C-terminus of H-CDR2 and the N-terminus of H-CDR3, and FW4 is the subsequence between the C-terminus of H-CDR3 and the C-terminus of the VH. Similarly, for a particular VL, FW1 is the subsequence between the N-terminus of the VL and the N-terminus of L-CDR1, FW2 is the subsequence between the C-terminus of L-CDR1 and the N-terminus of L-CDR2. FW3 is the subsequence between the C-terminus of L-CDR2 and the N-terminus of L-CDR3, and FW4 is the subsequence between the C-terminus of L-CDR3 and the C-terminus of the VL.

The variable domains of the heavy and light chains contain a region that interacts with a binding target, and this region interacting with a binding target is also referred to as an "antigen-binding site" or "antigen binding site" herein. The constant domains of the antibodies can mediate the binding of the antibody to host tissues or factors, including various cells of the immune system (e.g., effector cells) and the first component (C1q) of the classical complement system. Exemplary antibodies of the present disclosure include typical antibodies, but also bivalent fragments and variations thereof such as a F(ab')2.

As used herein, the term "antibody" encompasses intact polyclonal antibodies, intact monoclonal antibodies, bivalent antibody fragments (such as F(ab')2), multispecific antibodies such as bispecific antibodies, chimeric antibodies, humanized antibodies, human antibodies, and any other modified immunoglobulin molecule comprising an antigen binding site.

The term "humanized antibody" refers to forms of antibodies that contain sequences from non-human (eg, murine) antibodies as well as human antibodies. A humanized antibody can include conservative amino acid substitutions or non-natural residues from the same or different species that do not significantly alter its binding and/or biologic activity. Such antibodies are chimeric antibodies that contain minimal sequence derived from non-human immunoglobulins. For the most part, humanized antibodies are human immunoglobulins (recipient antibody) in which residues from a complementary-determining region (CDR) of the recipient are replaced by residues from a CDR of a non-human species (donor antibody) such as mouse, rat, camel, bovine, goat, or rabbit having the desired properties. Furthermore, humanized antibodies can comprise residues that are found neither in the recipient antibody nor in the imported CDR or framework sequences. These modifications are made to further refine and maximize antibody performance. Thus, in general, a humanized antibody can comprise all or substantially all of at least one, and in one aspect two, variable domains, in which all or substantially all of the hypervariable loops correspond to those of a non-human immunoglobulin and all or substantially all of the FR regions are those of a human immunoglobulin sequence. The humanized antibody optionally also can comprise at least a portion of an immunoglobulin constant region (Fc), or that of a human immunoglobulin. The process to humanize an antibody comprises a CDR-grafting technique. This technique implies the use of a "donor" antibody containing the antigen-specific CDRs and the "acceptor" antibody that will serve as the framework for the hybrid molecule. Usually, the "donor" antibody is the non-human derived antibody, such as the murine antibody, and the "acceptor" antibody is the human antibody. As a result of the humanization process, the resulting humanized antibody or hybrid antibody maintains the CDR sequences, in both the VL and VH domains, of the "donor" antibody, whereas the framework sequences are changed for those of the "acceptor" antibody.

An antibody can be of any of the five major classes (isotypes) of immunoglobulins: IgA, IgD, IgE, IgG, and IgM, or subclasses thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulins have different and well-known subunit structures and three-dimensional configurations. Antibodies can be naked or conjugated to other molecules such as therapeutic agents or diagnostic agents to form immunoconjugates.

The term "anticalin" refers to a protein that is derived from the lipocalin and that been engineered to bind to a specific target (see Skerra, 2008. FEBS J. 275(11):2677-83).

The term "antigen-binding fragment" or "Fab" refers to an antibody fragment comprising one constant and one variable domain of each of the heavy and light chain. A Fab fragment may be obtained by digesting an intact monoclonal antibody with papain.

The term "cancer" refers to a group of diseases, which can be defined as any abnormal benign or malignant new growth of tissue that possesses no physiological function and arises from uncontrolled usually rapid cellular proliferation and has the potential to invade or spread to other parts of the body.

"IL13Rα2-positive" cancer, including a "IL13Rα2-positive" cancerous disease, is one comprising cells, which have IL13Rα2 present at their cell surface. The term "IL13Rα2-positive" also refers to a cancer that produces sufficient levels of IL13Rα2 at the surface of cells thereof, such that a CAR-comprising cell of the present invention has a therapeutic effect, mediated by the binding of the CAR to IL13Rα2. In some embodiments, the IL13Rα2-positive cancer is glioblastoma or diffuse intrinsic pontine glioma.

The term "IL13Rα2-targeting moiety" refers to a substance that is able to bind IL13Rα2, preferably human IL13Rα2. Within the context of a CAR, a IL13Rα2-targeting moiety redirects T cells towards a IL13Rα2-positive cell, preferably a cancer cell. Within the context of a CAR, it is to be understood that the IL13Rα2-targeting moiety is genetically encodable.

The term "chimeric antigen receptor" or "CAR" refers to a synthetic receptor that targets T cells to a chosen antigen and reprograms T cell function, metabolism, and persistence. Similarly, the term "CART" refers to a T cell that comprises a CAR.

A "complete response" or "complete remission" or "CR" indicates the disappearance of all target lesions as defined in the RECIST v1.1 guideline. This does not always mean the cancer has been cured.

The term "costimulatory signaling domain" refers to a signaling moiety that provides to T cells a signal which, in addition to the primary signal provided by, for instance, the CD3ζ chain of the TCR/CD3 complex, mediates a T cell response, including, but not limited to, activation, proliferation, differentiation, cytokine secretion, and the like. A co-stimulatory domain can include all or a portion of, but is not limited to, CD27, CD28, 4-1BB (CD137), OX40 (CD134), CD30, CD40, ICOS, lymphocyte function-associated antigen-1 (LFA-1), CD2, IL13Rα2, LIGHT, NKG2C, B7-H3, and a ligand that specifically binds with CD83. In some embodiments, the co-stimulatory signaling domain is an intracellular signaling domain that interacts with other intracellular mediators to mediate a cell response including activation, proliferation, differentiation and cytokine secretion, and the like.

"Disease free survival" (DFS) refers to the length of time during and after treatment that the patient remains free of disease.

As used herein, the term "effective amount" of an agent, e.g., a therapeutic agent such as a CART, is that amount sufficient to effect beneficial or desired results, for example, clinical results, and, as such, an "effective amount" depends upon the context in which it is being applied. For example, in the context of administering a therapeutic agent that treats GBM or DIPG, an effective amount can reduce the number of cancer cells; reduce the tumor size or burden; inhibit (i.e., slow to some extent and in a certain embodiment, stop) cancer cell infiltration into peripheral organs; inhibit (i.e., slow to some extent and in a certain embodiment, stop) tumor metastasis; inhibit, to some extent, tumor growth; relieve to some extent one or more of the symptoms associated with the cancer; and/or result in a favorable response such as increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof. The term "effective amount" can be used interchangeably with "effective dose," "therapeutically effective amount," or "therapeutically effective dose".

The term "fynomer" refers to a protein that is derived from the SH3 domain of human Fyn kinase that has been engineered to bind to a specific target (see Bertschinger et al., 2007. Protein Eng Des Sel. 20(2):57-68).

The terms "individual", "patient" or "subject" are used interchangeably in the present application to designate a human being and are not meant to be limiting in any way. The "individual", "patient" or "subject" can be of any age, sex and physical condition. The term "patient in need thereof" usually refers to a patient who suffers from a IL13Rα2-positive cancer.

"Infusion" or "infusing" refers to the introduction of a therapeutic agent-containing solution into the body through a vein for therapeutic purposes. Generally, this is achieved via an intravenous bag.

"Intracellular signaling domain" as used herein refers to all or a portion of one or more domains of a molecule (here the chimeric antigen receptor molecule) that provides for activation of a T lymphocyte. Intracellular domains of such molecules mediate a signal by interacting with cellular mediators to result in proliferation, differentiation, activation and other effector functions. Examples of intracellular signaling domains for use in a CAR of the invention include the intracellular sequences of the CD3ζ chain, and/or co-receptors that act in concert to initiate signal transduction following CAR engagement, as well as any derivative or variant of these sequences and any synthetic sequence that has the same functional capability. T cell activation can be said to be mediated by two distinct classes of cytoplasmic signaling sequence: those that initiate antigen-dependent primary activation and provide a T cell receptor like signal (primary cytoplasmic signaling sequences) and those that act in an antigen-independent manner to provide a secondary or co-stimulatory signal (secondary cytoplasmic signaling sequences). Primary cytoplasmic signaling sequences that act in a stimulatory manner may contain signaling motifs which are known as receptor tyrosine-based activation motifs or ITAMs. Examples of ITAM containing primary cytoplasmic signaling sequences include those derived from CD3ζ, FcRy, CD3γ, CD3δ, CD3ε, CD5, CD22, CD79a, CD79b, and CD66d.

The term "monobody" refers to a protein that is derived from a fibronectin type III domain that has been engineered to bind to a specific target (see Koide et al., 2013. J Mol Biol. 415(2): 393-405).

The term "nanobody" refers to a protein comprising the soluble single antigen-binding V-domain of a heavy chain antibody, preferably a camelid heavy chain antibody (see Bannas et al., 2017. Front Immunol. 8:1603).

"Overall Survival" (OS) refers to the time from patient enrolment to death or censored at the date last known alive. OS includes a prolongation in life expectancy as compared to naive or untreated individuals or patients. Overall survival refers to the situation wherein a patient remains alive for a defined period of time, such as one year, five years, etc., e.g., from the time of diagnosis or treatment.

A "partial response" or "PR" refers to at least a 30% decrease in the sum of diameters of target lesions, taking as reference the baseline sum diameter, in response to treatment, as defined in the RECIST v1.1 guideline.

The term "peptide aptamer" refers to a short, 5-20 amino acid residue sequence that can bind to a specific target. Peptide aptamers are typically inserted within a loop region of a stable protein scaffold (see Reverdatto et al., 2015. Curr Top Med Chem. 15(12):1082-101).

As used herein, "pharmaceutically acceptable carrier" or "pharmaceutically acceptable diluent" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thiosulfate; low molecular weight proteins, such as human serum albumin, bovine serum albumin, gelatin, or other immunoglobulins; and hydrophilic polymers, such as polyvinylpyrrolidone.

"Progressive disease" or "disease that has progressed" refers to the appearance of one more new lesions or tumors and/or the unequivocal progression of existing non-target lesions as defined in the RECIST v1.1 guideline. Progressive disease or disease that has progressed can also refer to a tumor growth of more than 20 percent since treatment began, either due to an increase in mass or in spread of the tumor.

"Progression free survival" (PFS) refers to the time from enrolment to disease progression or death. PFS is generally measured using the Kaplan-Meier method and Response Evaluation Criteria in Solid Tumors (RECIST) 1.1 standards. Generally, progression free survival refers to the situation wherein a patient remains alive, without the cancer getting worse.

The term "RECIST" means Response Evaluation Criteria in Solid Tumours. RECIST guideline, criteria, or standard, describes a standard approach to solid tumor measurement and definitions for objective assessment of change in tumor size for use in adult and pediatric cancer clinical trials. RECIST v1.1 means version 1.1 of the revised RECIST guideline, and it is published in European Journal of Cancers 45 (2009) 228-247.

The term "repebody" refers to a protein that is derived from a leucine-rich repeat module and that been engineered to bind to a specific target (see Lee et al., 2012. PNAS. 109(9): 3299-3304).

The term "respond favorably" generally refers to causing a beneficial state in a subject. With respect to cancer treatment, the term refers to providing a therapeutic effect on the subject. Positive therapeutic effects in cancer can be measured in a number of ways. For example, tumor growth inhibition, molecular marker expression, serum marker expression, and molecular imaging techniques can all be used to assess therapeutic efficacy of an anti-cancer therapeutic. A favorable response can be assessed, for example, by increased progression-free survival (PFS), disease-free survival (DFS), or overall survival (OS), complete response (CR), partial response (PR), or, in some cases, stable disease (SD), a decrease in progressive disease (PD), a reduced time to progression (TTP) or any combination thereof.

"Percent (%) sequence identity" with respect to the proteins or nucleic acids described herein is defined as the percentage of amino acid residues or nucleobases, in a candidate sequence that are identical with the amino acid residues or nucleobases, respectively, in the reference sequence (i.e., the protein or polypeptide or nucleic acid from which it is derived), after aligning the sequences and introducing gaps, if necessary, to achieve the maximum percent sequence identity, and not considering any conservative substitutions as part of the sequence identity. Alignment for purposes of determining the percentage sequence identity can be achieved in various ways that are within the skill in the art, for example, using publicly available computer software such as BLAST. Those skilled in the art can determine appropriate parameters for measuring alignment, including any algorithms needed to achieve maximum alignment over the full-length or over a local region of the sequences being compared.

As explained above, the humanized versions of the murine scFvs are generated by maintaining the CDRs invariable or constant, while the framework are modified, namely humanized. By "maintained constant" it is referred that, while the framework regions of the targeting moiety can vary (for instance, they can be different depending on whether it is a murine scFv or a humanized scFv), the sequences belonging to the CDRs are not changed or are invariable.

In the context of the embodiments that refer to a percentage of identity over a VH or VL sequence where the CDRs of said regions are maintained constant, the percentage of identity at the time of comparing two sequences is obtained by aligning the sequences (e.g. VH sequences), preferably using BLAST tool (or a similar local alignment algorithm) and, once the percentage of identity is obtained, it is first confirmed (by, e.g. visual inspection) whether the specific CDRs are maintained constant between both sequences. In other words, to assess the percentage of identity between two sequences, both sequences are aligned over their entire length, and only the mismatches in the framework regions, and not in the CDRs, are considered to obtain the % of sequence identity between both sequences.

Preferably, the "percentage of sequence identity" as used herein is decided in the context of a local alignment, i.e., it is based on the alignment of regions of local similarity between sequences. Thus, in the context of the present invention, percentage of sequence identity is calculated preferably based on the local alignment comparison algorithm.

The term "single-chain antigen-binding fragment" or "scFab" refers to a fusion protein comprising one variable and one constant domain of the light chain of an antibody attached to one variable and one constant domain of the heavy chain of an antibody, wherein the heavy and light chains are linked together through a short peptide.

The term "single-chain variable fragment" or "scFv" refers to a fusion protein comprising the variable domains of the heavy chain and light chain of an antibody linked to one another with a peptide linker. The term also includes a disulfide stabilized Fv (dsFv).

"Stable disease" refers to disease without progression or relapse as defined in the RECIST v1.1 guideline. In stable disease there is neither sufficient tumor shrinkage to qualify for partial response, nor sufficient tumor increase to qualify as progressive disease.

"Time to Tumor Progression" (TTP) is defined as the time from enrolment to disease progression. TTP is generally measured using the RECIST v1.1 criteria.

The term "treatment" as used in the present application, refers to a set of hygienic, pharmacological, surgical and/or physical means used with the intent to cure and/or alleviate a disease and/or symptoms with the goal of remediating the health problem. This term includes curing the disease but also ameliorating, mitigating, or reducing the symptoms of said disease, including reducing the tumor burden or progression. Thus, the term "therapeutic treatment" or "treatment" as used herein refers to bringing a body from a pathological state or disease back to its normal, healthy state. The term "therapeutic treatment" or "treatment" as used herein, also refers to the mitigation, amelioration, or reduction of the harmful effect of a tumour, preferably a IL13Rα2 positive tumor, in a subject, or in some manner reducing the symptoms associated with such effects. The term "prophylactic treatment" as used herein refers to preventing a pathological state such as the development of a cancer.

### DESCRIPTION OF THE EMBODIMENTS

### IL13Rα2 targeting moieties of the invention.

As shown in the Examples below, four second-generation CARs anti-IL13Rα2 were generated. Transduction of T lymphocytes with lentiviral vectors resulted in an efficient expression of the four CARIL13Rα2 constructs (Fig. 3B). The four CARIL13Rα2 constructs also exhibited potent cytotoxicity against the GBM derived U87MG cell line after 24 hours, as determined by a luciferase-based assay (Fig. 5A). Fig. 6 also shows the ability of said CARs to mediate T cell activation, as measured by the levels of three proinflammatory cytokines.

Next, CARIL13Ra2_6A and CARIL13Rα2_46A were assayed *in vivo.* Although both CARIL13Rα2 T cells restricted the tumor volume in comparison to the UT T cells (Fig. 14B), only the robust antitumor activity of CARIL13Rα2_6A CAR T cells led to a significant improvement in the survival of mice treated with these cells compared to the control group (Fig. 14C). However, the CARIL13Rα2_46A CAR T cells showed a lower capacity to eradicate the tumor in this *in vivo* model. These results indicate that CARIL13Ra2_6A is more efficient in treating the tumor.

With the aim of assessing whether the CARIL13Ra2_6A and CARIL13Rα2_46A T cells are able to avoid tumor development before the U87MG engraftment, NSG mice were injected subcutaneously with 1×10⁶ U87MG GFP-LUC cells, and 24 hours later animals were treated with a single injection of 3×10⁶ CARIL13Rα2_6A+ or CARIL13Rα2_46A+ T cells (Fig. 17A). While CARIL13Rα2_46A T cells were able to control tumor progression, any remarkable effect was seen for CARIL13Ra2_6A T cells in comparison to the UT control T cells (Fig. 17B). This difference in the antitumor effect led to an improvement in the survival of mice treated with CARIL13Rα2_46A+ T cells (Fig. 17C). These results indicate that CARIL13Rα2_46A is more efficient in the prevention of tumor.

In view of the above, a **first aspect** of the present invention relates to a IL13Rα2 targeting moiety, also called herein IL13Rα2_6A, comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO: 1, or a sequence with at least 85%, 86%, 87%, 88%, 89%, 90%, 91%, 92%, 93%, 94%, 95%, 96%, 97%, 98%, 99% or 100% sequence identity to SEQ ID NO: 1;
- LCDR2 comprises, consists, or consists essentially of SEQ ID NO: 2, or a sequence with at least 85%, 86%, 87%, 88%, 89%, 90%, 91%, 92%, 93%, 94%, 95%, 96%, 97%, 98%, 99% or 100% sequence identity to SEQ ID NO: 2;
- LCDR3 comprises, consists, or consists essentially of SEQ ID NO: 3, or a sequence with at least 85%, 86%, 87%, 88%, 89%, 90%, 91%, 92%, 93%, 94%, 95%, 96%, 97%, 98%, 99% or 100% sequence identity to SEQ ID NO: 3;
- HCDR1 comprises, consists, or consists essentially of SEQ ID NO: 4, or a sequence with at least 85%, 86%, 87%, 88%, 89%, 90%, 91%, 92%, 93%, 94%, 95%, 96%, 97%, 98%, 99% or 100% sequence identity to SEQ ID NO: 4;
- HCDR2 comprises, consists, or consists essentially of SEQ ID NO: 5, or a sequence with at least 85%, 86%, 87%, 88%, 89%, 90%, 91%, 92%, 93%, 94%, 95%, 96%, 97%, 98%, 99% or 100% sequence identity to SEQ ID NO: 5; and
- HCDR3 comprises, consists, or consists essentially SEQ ID NO: 6, or a sequence with at least 85%, 86%, 87%, 88%, 89%, 90%, 91%, 92%, 93%, 94%, 95%, 96%, 97%, 98%, 99% or 100% sequence identity to SEQ ID NO: 6.

In some embodiments of the first aspect, the IL13Rα2_6A targeting moiety is an antibody, anticalin, repebody, monobody, preferably scFv, Fab, scFab, affibody, fynomer, DARPin, nanobody, or peptide aptamer that specifically binds to IL13Rα2.

In some embodiments of the first aspect, the IL13Rα2_6A targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 1, LCDR2 consists of SEQ ID NO: 2, LCDR3 consists of SEQ ID NO: 3, HCDR1 consists of SEQ ID NO: 4, HCDR2 consists of SEQ ID NO: 5, and HCDR3 consists of SEQ ID NO: 6.

Preferably, the IL13Rα2_6A targeting moiety of the first aspect is an antibody, F(ab')2, Fab, preferably scFv, or scFab comprising a VL domain and a VH domain, wherein the VL domain comprises, consists, or consists essentially of SEQ ID NO: 7, and wherein the VH domain comprises, consists, or consists essentially of SEQ ID NO: 8. In some embodiments, the IL13Rα2-targeting moiety of the first aspect is an antibody, preferably scFv, Fab, or scFab comprising a VL domain and VH domain, wherein the VL domain comprises SEQ ID NO: 7 and the VH domain comprises SEQ ID NO: 8. In some embodiments, the IL13Rα2-targeting moiety of the first aspect is an antibody, preferably scFv, Fab, or scFab comprising a VL domain and VH domain, wherein the VL domain consists of SEQ ID NO: 7 and the VH domain consists of SEQ ID NO: 8.

The VL and VH of the IL13Rα2_6A targeting moiety described herein in the first aspect, or any of its embodiments, may be humanized. In the humanization process, the framework regions of the variable region of a targeting moiety may be modified, whereas the CDRs are maintained constant. Therefore, in some embodiments of the first aspect, the IL13Rα2_6A targeting moiety is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 7 or 9, preferably SEQ ID NO: 7, wherein the CDRs comprised in said VL are maintained constant (i.e., non-variable) and consists of SEQ ID NOs: 1, 2, and 3; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 8 or 10, preferably SEQ ID NO: 8, wherein the CDRs comprised in said VH are maintained constant and consist of SEQ ID NOs: 4, 5, and 6.

Thus, in an embodiment of the first aspect, the IL13Rα2_6A targeting moiety is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 7 or 9, preferably SEQ ID NO: 7, and wherein said VL domain comprises CDR polypeptides consisting of LCDR1 as set forth in SEQ ID NO: 1, LCDR2 as set forth in SEQ ID NO: 2, LCDR3 as set forth in SEQ ID NO: 3, and
- said VH domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 8 or 10, preferably SEQ ID NO: 8, and wherein said VH domain comprises CDR polypeptides consisting of HCDR1 as set forth in SEQ ID NO: 4, HCDR2 as set forth in SEQ ID NO: 5, and HCDR3 as set forth in SEQ ID NO: 6 and
preferably, wherein the IL13Rα2_6A targeting moiety is capable of binding to human IL13Rα2, preferably as measured by surface plasmon resonance (SPR).

In an embodiment of the first aspect, the IL13Rα2_6A targeting moiety is an antibody, F(ab')2, Fab, preferably scFv, or scFab, and is capable of binding to human IL13Rα2 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 7, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 8, and
wherein the LCDR1, 2, and 3 comprised in the VL domain consist of SEQ ID NO: 1, 2 and 3, respectively, and wherein the HCDR1, 2 and 3 comprised in said VH domain consists of SEQ ID NO: 4, 5 and 6, respectively.

Preferably, the IL13Rα2_6A targeting moiety of the first aspect is an antibody, F(ab')2, Fab, preferably scFv, or scFab comprising a VL domain and a VH domain, wherein the VL domain comprises, consists, or consists essentially of SEQ ID NO: 9, and wherein the VH domain comprises, consists, or consists essentially of SEQ ID NO: 10. In some embodiments, the IL13Rα2_6A targeting moiety of the first aspect is an scFv comprising a VL domain and VH domain, wherein the VL domain comprises SEQ ID NO: 9 and the VH domain comprises SEQ ID NO: 10. In some embodiments, the IL13Rα2_6A targeting moiety of the first aspect is an scFv comprising a VL domain and VH domain, wherein the VL domain consists of SEQ ID NO: 9 and the VH domain consists of SEQ ID NO: 10.

Preferably, the IL13Rα2_6A targeting moiety of the first aspect comprises, consists, or consists essentially of SEQ ID NO: 13 or 15, preferably SEQ ID NO: 13, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 13 or 15, respectively, preferably SEQ ID NO: 13. Most preferably, the IL13Rα2_6A targeting moiety consists of SEQ ID NO: 13 or 15, preferably SEQ ID NO: 13.

A second aspect of the present invention relates to a IL13Rα2 targeting moiety, also called herein IL13Rα2_46A, comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO: 17, or a sequence with at least 85%, 86%, 87%, 88%, 89%, 90%, 91%, 92%, 93%, 94%, 95%, 96%, 97%, 98%, 99% or 100% sequence identity to SEQ ID NO: 17;
- LCDR2 comprises, consists, or consists essentially of SEQ ID NO: 18, or a sequence with at least 85%, 86%, 87%, 88%, 89%, 90%, 91%, 92%, 93%, 94%, 95%, 96%, 97%, 98%, 99% or 100% sequence identity to SEQ ID NO: 18;
- LCDR3 comprises, consists, or consists essentially of SEQ ID NO: 19, or a sequence with at least 85%, 86%, 87%, 88%, 89%, 90%, 91%, 92%, 93%, 94%, 95%, 96%, 97%, 98%, 99% or 100% sequence identity to SEQ ID NO: 19;
- HCDR1 comprises, consists, or consists essentially of SEQ ID NO: 20, or a sequence with at least 85%, 86%, 87%, 88%, 89%, 90%, 91%, 92%, 93%, 94%, 95%, 96%, 97%, 98%, 99% or 100% sequence identity to SEQ ID NO: 20;
- HCDR2 comprises, consists, or consists essentially of SEQ ID NO: 21, or a sequence with at least 85%, 86%, 87%, 88%, 89%, 90%, 91%, 92%, 93%, 94%, 95%, 96%, 97%, 98%, 99% or 100% sequence identity to SEQ ID NO: 21; and
- HCDR3 comprises, consists, or consists essentially SEQ ID NO: 22, or a sequence with at least 85%, 86%, 87%, 88%, 89%, 90%, 91%, 92%, 93%, 94%, 95%, 96%, 97%, 98%, 99% or 100% sequence identity to SEQ ID NO: 22.

In some embodiments of the second aspect, the IL13Rα2_46A targeting moiety is an antibody, anticalin, repebody, monobody, preferably scFv, Fab, scFab, affibody, fynomer, DARPin, nanobody, or peptide aptamer that specifically binds to IL13Rα2.

In some embodiments of the second aspect, the IL13Rα2_46A targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 17, LCDR2 consists of SEQ ID NO: 18, LCDR3 consists of SEQ ID NO: 19, HCDR1 consists of SEQ ID NO: 20, HCDR2 consists of SEQ ID NO: 21, and HCDR3 consists of SEQ ID NO: 22.

Preferably, the IL13Rα2_46A targeting moiety of the second aspect is an antibody, F(ab')2, Fab, preferably scFv, or scFab comprising a VL domain and a VH domain, wherein the VL domain comprises, consists, or consists essentially of SEQ ID NO: 23; and wherein the VH domain comprises, consists, or consists essentially of SEQ ID NO: 24. In some embodiments, the IL13Rα2-targeting moiety of the second aspect is an antibody, preferably scFv, Fab, or scFab comprising a VL domain and VH domain, wherein the VL domain comprises SEQ ID NO: 23 and the VH domain comprises SEQ ID NO: 24. In some embodiments, the IL13Rα2-targeting moiety of the second aspect is an antibody, preferably scFv, Fab, or scFab comprising a VL domain and VH domain, wherein the VL domain consists of SEQ ID NO: 23 and the VH domain consists of SEQ ID NO: 24.

The VL and VH of the IL13Rα2_46A targeting moiety described herein in the second aspect, or any of its embodiments, may be humanized. In the humanization process, the framework regions of the variable region of a targeting moiety may be modified, whereas the CDRs are maintained constant. Therefore, in some embodiments of the second aspect, the IL13Rα2_46A targeting moiety is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 23 or 25, preferably SEQ ID NO: 23, wherein the CDRs comprised in said VL are maintained constant (i.e., non-variable) and consists of SEQ ID NOs: 17, 18 and 19; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 24 or 26, preferably SEQ ID NO: 24, wherein the CDRs comprised in said VH are maintained constant and consist of SEQ ID NOs: 20, 21 and 22.

Thus, in an embodiment of the second aspect, the IL13Rα2_46A targeting moiety is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 23 or 25, preferably SEQ ID NO: 23, and wherein said VL domain comprises CDR polypeptides consisting of LCDR1 as set forth in SEQ ID NO: 17, LCDR2 as set forth in SEQ ID NO: 18, LCDR3 as set forth in SEQ ID NO: 19, and
- said VH domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 24 or 26, preferably SEQ ID NO: 24, and wherein said VH domain comprises CDR polypeptides consisting of HCDR1 as set forth in SEQ ID NO: 20, HCDR2 as set forth in SEQ ID NO: 21, and HCDR3 as set forth in SEQ ID NO: 22 and
preferably, wherein the IL13Rα2_46A targeting moiety is capable of binding to human IL13Rα2, preferably as measured by surface plasmon resonance (SPR).

In an embodiment of the second aspect, the IL13Rα2_46A targeting moiety is an antibody, F(ab')2, Fab, preferably scFv, or scFab, and is capable of binding to human IL13Rα2 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 23, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 24, and
wherein the LCDR1, 2, and 3 comprised in the VL domain consist of SEQ ID NO: 17, 18 and 19, respectively, and wherein the HCDR1, 2 and 3 comprised in said VH domain consists of SEQ ID NO: 20, 21 and 22 respectively.

Preferably, the IL13Rα2_46A targeting moiety of the second aspect is an antibody, F(ab')2, Fab, preferably scFv, or scFab comprising a VL domain and a VH domain, wherein the VL domain comprises, consists, or consists essentially of SEQ ID NO: 25; and wherein the VH domain comprises, consists, or consists essentially of SEQ ID NO: 26. In some embodiments, the IL13Rα2_46A targeting moiety of the second aspect is an scFv comprising a VL domain and VH domain, wherein the VL domain comprises SEQ ID NO: 25 and the VH domain comprises SEQ ID NO: 26. In some embodiments, the IL13Rα2_46A targeting moiety of the second aspect is an scFv comprising a VL domain and VH domain, wherein the VL domain consists of SEQ ID NO: 25 and the VH domain consists of SEQ ID NO: 26.

Preferably, the IL13Rα2_46A targeting moiety of the second aspect comprises, consists, or consists essentially of SEQ ID NO: 29 or 31, preferably SEQ ID NO: 29, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 29 or 31, respectively, preferably SEQ ID NO: 29. Most preferably, the IL13Rα2_46A targeting moiety consists of SEQ ID NO: 29 or 31, preferably SEQ ID NO: 29.

A **third aspect** of the present invention relates to a IL13Rα2 targeting moiety, also called herein IL13Rα2_143A, comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO: 33, or a sequence with at least 85%, 86%, 87%, 88%, 89%, 90%, 91%, 92%, 93%, 94%, 95%, 96%, 97%, 98%, 99% or 100% sequence identity to SEQ ID NO: 33
- LCDR2 comprises, consists, or consists essentially of SEQ ID NO: 34, or a sequence with at least 85%, 86%, 87%, 88%, 89%, 90%, 91%, 92%, 93%, 94%, 95%, 96%, 97%, 98%, 99% or 100% sequence identity to SEQ ID NO: 34;
- LCDR3 comprises, consists, or consists essentially of SEQ ID NO: 35, or a sequence with at least 85%, 86%, 87%, 88%, 89%, 90%, 91%, 92%, 93%, 94%, 95%, 96%, 97%, 98%, 99% or 100% sequence identity to SEQ ID NO: 35;
- HCDR1 comprises, consists, or consists essentially of SEQ ID NO: 36, or a sequence with at least 85%, 86%, 87%, 88%, 89%, 90%, 91%, 92%, 93%, 94%, 95%, 96%, 97%, 98%, 99% or 100% sequence identity to SEQ ID NO: 36;
- HCDR2 comprises, consists, or consists essentially of SEQ ID NO: 37, or a sequence with at least 85%, 86%, 87%, 88%, 89%, 90%, 91%, 92%, 93%, 94%, 95%, 96%, 97%, 98%, 99% or 100% sequence identity to SEQ ID NO: 37; and
- HCDR3 comprises, consists, or consists essentially SEQ ID NO: 38, or a sequence with at least 85%, 86%, 87%, 88%, 89%, 90%, 91%, 92%, 93%, 94%, 95%, 96%, 97%, 98%, 99% or 100% sequence identity to SEQ ID NO: 38.

In some embodiments of the third aspect, the IL13Rα2_143A targeting moiety is an antibody, anticalin, repebody, monobody, preferably scFv, Fab, scFab, affibody, fynomer, DARPin, nanobody, or peptide aptamer that specifically binds to IL13Rα2.

In some embodiments of the third aspect, the IL13Rα2_143A targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 33, LCDR2 consists of SEQ ID NO: 34, LCDR3 consists of SEQ ID NO: 35, HCDR1 consists of SEQ ID NO: 36, HCDR2 consists of SEQ ID NO: 37, and HCDR3 consists of SEQ ID NO: 38.

Preferably, the IL13Rα2_143A targeting moiety of the third aspect is an antibody, F(ab')2, Fab, preferably scFv, or scFab comprising a VL domain and a VH domain, wherein the VL domain comprises, consists, or consists essentially of SEQ ID NO: 39; and wherein the VH domain comprises, consists, or consists essentially of SEQ ID NO: 40. In some embodiments, the IL13Rα2-targeting moiety of the third aspect is an antibody, preferably scFv, Fab, or scFab comprising a VL domain and VH domain, wherein the VL domain comprises SEQ ID NO: 39 and the VH domain comprises SEQ ID NO: 40. In some embodiments, the IL13Rα2-targeting moiety of the third aspect is an antibody, preferably scFv, Fab, or scFab comprising a VL domain and VH domain, wherein the VL domain consists of SEQ ID NO: 39 and the VH domain consists of SEQ ID NO: 40.

The VL and VH of the IL13Rα2_143A targeting moiety described herein in the third aspect, or any of its embodiments, may be humanized. In the humanization process, the framework regions of the variable region of a targeting moiety may be modified, whereas the CDRs are maintained constant. Therefore, in some embodiments of the third aspect, the IL13Rα2_143A targeting moiety is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 39 or 41, preferably SEQ ID NO: 39, wherein the CDRs comprised in said VL are maintained constant (i.e., non variable) and consists of SEQ ID NOs: 33, 34 and 35; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 40 or 42, preferably SEQ ID NO: 40, wherein the CDRs comprised in said VH are maintained constant and consist of SEQ ID NOs: 36, 37 and 38.

Thus, in an embodiment of the third aspect, the IL13Rα2_143A targeting moiety is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 39 or 41, preferably SEQ ID NO: 39, and wherein said VL domain comprises CDR polypeptides consisting of LCDR1 as set forth in SEQ ID NO: 33, LCDR2 as set forth in SEQ ID NO: 34, LCDR3 as set forth in SEQ ID NO: 35, and
- said VH domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 40 or 42, preferably SEQ ID NO: 40, and wherein said VH domain comprises CDR polypeptides consisting of HCDR1 as set forth in SEQ ID NO: 36, HCDR2 as set forth in SEQ ID NO: 37, and HCDR3 as set forth in SEQ ID NO: 38 and
preferably, wherein the IL13Rα2_143A targeting moiety is capable of binding to human IL13Rα2, preferably as measured by surface plasmon resonance (SPR).

In an embodiment of the third aspect, the IL13Rα2_143A targeting moiety is an antibody, F(ab')2, Fab, preferably scFv, or scFab, and is capable of binding to human IL13Rα2 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 39, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 40, and
wherein the LCDR1, 2, and 3 comprised in the VL domain consist of SEQ ID NO: 33, 34 and 35, respectively, and wherein the HCDR1, 2 and 3 comprised in said VH domain consists of SEQ ID NO: 36, 37 and 38 respectively.

Preferably, the IL13Rα2_143A targeting moiety of the third aspect is an antibody, F(ab')2, Fab, preferably scFv, or scFab comprising a VL domain and a VH domain, wherein the VL domain comprises, consists, or consists essentially of SEQ ID NO: 41; and wherein the VH domain comprises, consists, or consists essentially of SEQ ID NO: 42. In some embodiments, the IL13Rα2_143A targeting moiety of the third aspect is an scFv comprising a VL domain and VH domain, wherein the VL domain comprises SEQ ID NO: 41 and the VH domain comprises SEQ ID NO: 42. In some embodiments, the IL13Rα2_143A targeting moiety of the third aspect is an scFv comprising a VL domain and VH domain, wherein the VL domain consists of SEQ ID NO: 41 and the VH domain consists of SEQ ID NO: 42.

Preferably, the IL13Rα2_143A targeting moiety of the third aspect comprises, consists, or consists essentially of SEQ ID NO: 45 or 47, preferably SEQ ID NO: 45, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 45 or 47, respectively, preferably SEQ ID NO: 45. Most preferably, the IL13Rα2_143A targeting moiety consists of SEQ ID NO: 45 or 47, preferably SEQ ID NO: 45.

A **fourth aspect** of the present invention relates to a IL13Rα2 targeting moiety, also called herein IL13Rα2_82B, comprising a VL domain and a VH domain, wherein said VL domain comprises LCDR1, LCDR2 and LCDR3 polypeptides and said VH domain comprises HCDR1, HCDR2 and HCDR3 polypeptides, and wherein:
- LCDR1 comprises, consists, or consists essentially of SEQ ID NO: 49, or a sequence with at least 85%, 86%, 87%, 88%, 89%, 90%, 91%, 92%, 93%, 94%, 95%, 96%, 97%, 98%, 99% or 100% sequence identity to SEQ ID NO: 49;
- LCDR2 comprises, consists, or consists essentially of SEQ ID NO: 50, or a sequence with at least 85%, 86%, 87%, 88%, 89%, 90%, 91%, 92%, 93%, 94%, 95%, 96%, 97%, 98%, 99% or 100% sequence identity to SEQ ID NO: 50;
- LCDR3 comprises, consists, or consists essentially of SEQ ID NO: 51, or a sequence with at least 85%, 86%, 87%, 88%, 89%, 90%, 91%, 92%, 93%, 94%, 95%, 96%, 97%, 98%, 99% or 100% sequence identity to SEQ ID NO: 51;
- HCDR1 comprises, consists, or consists essentially of SEQ ID NO: 52, or a sequence with at least 85%, 86%, 87%, 88%, 89%, 90%, 91%, 92%, 93%, 94%, 95%, 96%, 97%, 98%, 99% or 100% sequence identity to SEQ ID NO: 52;
- HCDR2 comprises, consists, or consists essentially of SEQ ID NO: 53, or a sequence with at least 85%, 86%, 87%, 88%, 89%, 90%, 91%, 92%, 93%, 94%, 95%, 96%, 97%, 98%, 99% or 100% sequence identity to SEQ ID NO: 53; and
- HCDR3 comprises, consists, or consists essentially SEQ ID NO: 54, or a sequence with at least 85%, 86%, 87%, 88%, 89%, 90%, 91%, 92%, 93%, 94%, 95%, 96%, 97%, 98%, 99% or 100% sequence identity to SEQ ID NO: 54.

In some embodiments of the fourth aspect, the IL13Rα2_82B targeting moiety is an antibody, anticalin, repebody, monobody, preferably scFv, Fab, scFab, affibody, fynomer, DARPin, nanobody, or peptide aptamer that specifically binds to IL13Rα2.

In some embodiments of the fourth aspect, the IL13Rα2_82B targeting moiety is an antibody, F(ab')2, Fab, preferably scFv, or scFab comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 49, LCDR2 consists of SEQ ID NO: 50, LCDR3 consists of SEQ ID NO: 51, HCDR1 consists of SEQ ID NO: 52, HCDR2 consists of SEQ ID NO: 53, and HCDR3 consists of SEQ ID NO: 54.

Preferably, the IL13Rα2_82B targeting moiety of the fourth aspect is an antibody, F(ab')2, Fab, preferably scFv, or scFab comprising a VL domain and a VH domain, wherein the VL domain comprises, consists, or consists essentially of SEQ ID NO: 55; and wherein the VH domain comprises, consists, or consists essentially of SEQ ID NO: 56. In some embodiments, the IL13Rα2-targeting moiety of the fourth aspect is an antibody, preferably scFv, Fab, or scFab comprising a VL domain and VH domain, wherein the VL domain comprises SEQ ID NO: 55 and the VH domain comprises SEQ ID NO: 56. In some embodiments, the IL13Rα2-targeting moiety of the fourth aspect is an antibody, preferably scFv, Fab, or scFab comprising a VL domain and VH domain, wherein the VL domain consists of SEQ ID NO: 55 and the VH domain consists of SEQ ID NO: 56.

The VL and VH of the IL13Rα2_82B targeting moiety described herein in the fourth aspect, or any of its embodiments, may be humanized. In the humanization process, the framework regions of the variable region of a targeting moiety may be modified, whereas the CDRs are maintained constant. Therefore, in some embodiments of the fourth aspect, the IL13Rα2_82B targeting moiety is an antibody, F(ab')2, Fab, preferably scFv, or scFab, comprising a VL and a VH domains, wherein the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 55 or 57, preferably SEQ ID NO: 55, wherein the CDRs comprised in said VL are maintained constant (i.e., non-variable) and consists of SEQ ID NOs: 49, 50 and 51; and wherein the VH domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 56 or 58, preferably SEQ ID NO: 56, wherein the CDRs comprised in said VH are maintained constant and consist of SEQ ID NOs: 52, 53 and 54.

Thus, in an embodiment of the fourth aspect, the IL13Rα2_82B targeting moiety is an antibody, F(ab')2, Fab, preferably scFv, or scFab, and comprises a VL domain and a VH domain, wherein:
- said VL domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 55 or 57, preferably SEQ ID NO: 55, and wherein said VL domain comprises CDR polypeptides consisting of LCDR1 as set forth in SEQ ID NO: 49, LCDR2 as set forth in SEQ ID NO: 50, LCDR3 as set forth in SEQ ID NO: 51, and
- said VH domain comprises a sequence with at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 56 or 58, preferably SEQ ID NO: 56, and wherein said VH domain comprises CDR polypeptides consisting of HCDR1 as set forth in SEQ ID NO: 52, HCDR2 as set forth in SEQ ID NO: 53, and HCDR3 as set forth in SEQ ID NO: 54 and
preferably, wherein the IL13Rα2_82B targeting moiety is capable of binding to human IL13Rα2, preferably as measured by surface plasmon resonance (SPR).

In an embodiment of the fourth aspect, the IL13Rα2_82B targeting moiety is an antibody, F(ab')2, Fab, preferably scFv, or scFab, and is capable of binding to human IL13Rα2 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 55, and
- the VH domain comprising a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 56, and
wherein the LCDR1, 2, and 3 comprised in the VL domain consist of SEQ ID NO: 49, 50 and 51, respectively, and wherein the HCDR1, 2 and 3 comprised in said VH domain consists of SEQ ID NO: 52, 53 and 54 respectively.

Preferably, the IL13Rα2_82B targeting moiety of the fourth aspect is an antibody, F(ab')2, Fab, preferably scFv, or scFab comprising a VL domain and a VH domain, wherein the VL domain comprises, consists, or consists essentially of SEQ ID NO: 57; and wherein the VH domain comprises, consists, or consists essentially of SEQ ID NO: 58. In some embodiments, the IL13Rα2_82B targeting moiety of the fourth aspect is an scFv comprising a VL domain and VH domain, wherein the VL domain comprises SEQ ID NO: 57 and the VH domain comprises SEQ ID NO: 58. In some embodiments, the IL13Rα2_82B targeting moiety of the fourth aspect is an scFv comprising a VL domain and VH domain, wherein the VL domain consists of SEQ ID NO: 57 and the VH domain consists of SEQ ID NO: 58.

Preferably, the IL13Rα2_82B targeting moiety of the fourth aspect comprises, consists, or consists essentially of SEQ ID NO: 61 or63, preferably SEQ ID NO: 61, or a sequence that has at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 61 or 63, respectively, preferably SEQ ID NO: 61. Most preferably, the IL13Rα2_82B targeting moiety consists of SEQ ID NO: 61 or 63, preferably SEQ ID NO: 61.

In some embodiments, the VL and VH domains of the IL13Rα2 targeting moieties defined in the first, second, third, and fourth aspects, or any of their embodiments, are linked by a peptide linker. In some embodiments, the linker comprises at least 5 amino acids, preferably between 5 and 25, preferably between 10-20 amino acids, most preferably 20 amino acids. Preferably, the amino acid is G. Preferably, the peptide linker comprises, consists, or consists essentially of SEQ ID NO: 65, or a sequence that has 90%, 91%, 92%, 93%, 94%, 95%, 96%, 97%, 98%, or 100% sequence identity to SEQ ID NO: 65.

In a preferred embodiment, the IL13Rα2 targeting moieties defined in the first, second, third, and fourth aspects, or any of their embodiments, further comprise a signal peptide that is placed preferably at the N-terminal region of the IL13Rα2 targeting moiety. Preferably, the signal peptide comprises, consists, or consists essentially of SEQ ID NO: 66, or a sequence that has 90%, 91%, 92%, 93%, 94%, 95%, 96%, 97%, 98%, or 100% sequence identity to SEQ ID NO: 66.

The IL13Rα2 targeting moieties as defined above in the first, second, third, and fourth aspects, or any of their embodiments, can be part of a chimeric antigen receptor (CAR).

Thus, in a preferred embodiment, the present invention provides a chimeric antigen receptor (CAR) comprising:
a) an extracellular domain comprising a IL13Rα2 targeting moiety as defined above in the first, second, third, and fourth aspects, or any of their embodiments,
b) a transmembrane domain;
c) an intracellular signaling domain and,
d) optionally, a costimulatory domain.

Each of the elements of the CAR according to this embodiment are further developed below:
*a) extracellular domain comprising a IL13Rα2 targeting moiety* as *defined in the first, second, third, and fourth aspects, or any of their embodiments,*

In an embodiment the extracellular domain comprises at least one, preferably one, of the IL13Rα2 targeting moieties as defined above in the first to fourth aspects, or any of their embodiments. Thus, all the embodiments defined above in said aspects apply herein.

Preferably, the IL13Rα2 targeting moiety comprised in the extracellular domain is the targeting moiety as defined in the first aspect or any of its embodiments (IL13Rα2_6A). Preferably, the IL13Rα2 targeting moiety comprised in the extracellular domain is a scFv comprising a VL domain and VH domain, wherein said VL domain comprises LCDR1, LCDR2 and LCDR3 polypeptides and said VH domain comprises HCDR1, HCDR2 and HCDR3 polypeptides, and wherein said VL domain comprises LCDR1, LCDR2 and LCDR3 polypeptides and said VH domain comprises HCDR1, HCDR2 and HCDR3 polypeptides, and LCDR1 consists of SEQ ID NO: 1, LCDR2 consists of SEQ ID NO: 2, LCDR3 consists of SEQ ID NO: 3, HCDR1 consists of SEQ ID NO: 4, HCDR2 consists of SEQ ID NO: 5, and HCDR3 consists of SEQ ID NO: 6.

Preferably, the IL13Rα2 targeting moiety comprised in the extracellular domain is capable of binding to human IL13Rα2 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, preferably 85%, 86%, 87%, 88%, 89%, 90%, 91%, 92%, 93%, 94%, 95%, 96%, 97%, 98%, 99% or 100% sequence identity to SEQ ID NO: 7, and
- the VH domain comprising a sequence having at least 80%, 81%, 82%, 83%, 84%, preferably 85%, 86%, 87%, 88%, 89%, 90%, 91%, 92%, 93%, 94%, 95%, 96%, 97%, 98%, 99% or 100% sequence identity to SEQ ID NO: 8, and
wherein the LCDR1, 2, and 3 comprised in the VL domain consist of SEQ ID NO: 1, 2 and 3, respectively, and wherein the HCDR1, 2 and 3 comprised in said VH domain consists of SEQ ID NO: 4, 5 and 6, respectively.

Preferably, the IL13Rα2 targeting moiety comprised in the extracellular domain is a scFv comprising a VL domain and VH domain, wherein the VL domain comprises SEQ ID NO: 7 and the VH domain comprises SEQ ID NO: 8. Preferably, the IL13Rα2 targeting moiety comprised in the extracellular domain is a scFv comprising a VL domain and VH domain, wherein the VL domain consists of SEQ ID NO: 7 and the VH domain consists of SEQ ID NO: 8.

Preferably, the IL13Rα2 targeting moiety comprised in the extracellular domain is a scFv comprising a VL domain and VH domain, wherein the VL domain comprises SEQ ID NO: 9 and the VH domain comprises SEQ ID NO: 10. Preferably, the IL13Rα2 targeting moiety comprised in the extracellular domain is a scFv comprising a VL domain and VH domain, wherein the VL domain consists of SEQ ID NO: 9 and the VH domain consists of SEQ ID NO: 10.

Most preferably, the IL13Rα2 targeting moiety comprised in the extracellular domain is a scFv that comprises, consists, or consists essentially of SEQ ID NO: 13, or a sequence that has 90%, 91%, 92%, 93%, 94%, 95%, 96%, 97%, 98%, or 100% sequence identity to SEQ ID NO: 13. Most preferably, the IL13Rα2 targeting moiety is a scFv that consists of SEQ ID NO: 13. Most preferably, the IL13Rα2 targeting moiety comprised in the extracellular domain is a scFv that comprises, consists, or consists essentially of SEQ ID NO: 15, or a sequence that has 90%, 91%, 92%, 93%, 94%, 95%, 96%, 97%, 98%, or 100% sequence identity to SEQ ID NO: 15. Most preferably, the IL13Rα2 targeting moiety is a scFv that consists of SEQ ID NO: 15.

Preferably, the IL13Rα2 targeting moiety comprised in the extracellular domain is the targeting moiety as defined in the second aspect or any of its embodiments (IL13Rα2_46A). Preferably, the IL13Rα2 targeting moiety comprised in the extracellular domain is a scFv comprising a VL domain and VH domain, wherein said VL domain comprises LCDR1, LCDR2 and LCDR3 polypeptides and said VH domain comprises HCDR1, HCDR2 and HCDR3 polypeptides, and wherein said VL domain comprises LCDR1, LCDR2 and LCDR3 polypeptides and said VH domain comprises HCDR1, HCDR2 and HCDR3 polypeptides, and LCDR1 consists of SEQ ID NO: 17, LCDR2 consists of SEQ ID NO: 18, LCDR3 consists of SEQ ID NO: 19, HCDR1 consists of SEQ ID NO: 20, HCDR2 consists of SEQ ID NO: 21, and HCDR3 consists of SEQ ID NO: 22.

Preferably, the IL13Rα2 targeting moiety comprised in the extracellular domain is capable of binding to human IL13Rα2 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, preferably 85%, 86%, 87%, 88%, 89%, 90%, 91%, 92%, 93%, 94%, 95%, 96%, 97%, 98%, 99% or 100% sequence identity to SEQ ID NO: 23, and
- the VH domain comprising a sequence having at least 80%, 81%, 82%, 83%, 84%, preferably 85%, 86%, 87%, 88%, 89%, 90%, 91%, 92%, 93%, 94%, 95%, 96%, 97%, 98%, 99% or 100% sequence identity to SEQ ID NO: 24, and
wherein the LCDR1, 2, and 3 comprised in the VL domain consist of SEQ ID NO: 17, 18 and 19, respectively, and wherein the HCDR1, 2 and 3 comprised in said VH domain consists of SEQ ID NO: 20, 21 and 22, respectively.

Preferably, the IL13Rα2 targeting moiety comprised in the extracellular domain is a scFv comprising a VL domain and VH domain, wherein the VL domain comprises SEQ ID NO: 23 and the VH domain comprises SEQ ID NO: 24. Preferably, the IL13Rα2 targeting moiety comprised in the extracellular domain is a scFv comprising a VL domain and VH domain, wherein the VL domain consists of SEQ ID NO: 23 and the VH domain consists of SEQ ID NO: 24.

Preferably, the IL13Rα2 targeting moiety comprised in the extracellular domain is a scFv comprising a VL domain and VH domain, wherein the VL domain comprises SEQ ID NO: 25 and the VH domain comprises SEQ ID NO: 26. Preferably, the IL13Rα2 targeting moiety comprised in the extracellular domain is a scFv comprising a VL domain and VH domain, wherein the VL domain consists of SEQ ID NO: 25 and the VH domain consists of SEQ ID NO: 26.

Most preferably, the IL13Rα2 targeting moiety comprised in the extracellular domain is a scFv that comprises, consists, or consists essentially of SEQ ID NO: 29, or a sequence that has 90%, 91%, 92%, 93%, 94%, 95%, 96%, 97%, 98%, or 100% sequence identity to SEQ ID NO: 29. Most preferably, the IL13Rα2 targeting moiety is a scFv that consists of SEQ ID NO: 29. Most preferably, the IL13Rα2 targeting moiety comprised in the extracellular domain is a scFv that comprises, consists, or consists essentially of SEQ ID NO: 31, or a sequence that has 90%, 91%, 92%, 93%, 94%, 95%, 96%, 97%, 98%, or 100% sequence identity to SEQ ID NO: 31. Most preferably, the IL13Rα2 targeting moiety is a scFv that consists of SEQ ID NO: 31.

Preferably, the IL13Rα2 targeting moiety comprised in the extracellular domain is the targeting moiety as defined in the third aspect or any of its embodiments (IL13Rα2_143A). Preferably, the IL13Rα2 targeting moiety comprised in the extracellular domain is a scFv comprising a VL domain and VH domain, wherein said VL domain comprises LCDR1, LCDR2 and LCDR3 polypeptides and said VH domain comprises HCDR1, HCDR2 and HCDR3 polypeptides, and wherein said VL domain comprises LCDR1, LCDR2 and LCDR3 polypeptides and said VH domain comprises HCDR1, HCDR2 and HCDR3 polypeptides, and LCDR1 consists of SEQ ID NO: 33, LCDR2 consists of SEQ ID NO: 34, LCDR3 consists of SEQ ID NO: 35, HCDR1 consists of SEQ ID NO: 36, HCDR2 consists of SEQ ID NO: 37, and HCDR3 consists of SEQ ID NO: 38.

Preferably, the IL13Rα2 targeting moiety comprised in the extracellular domain is capable of binding to human IL13Rα2 and comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, preferably 85%, 86%, 87%, 88%, 89%, 90%, 91%, 92%, 93%, 94%, 95%, 96%, 97%, 98%, 99% or 100% sequence identity to SEQ ID NO: 39, and
- the VH domain comprising a sequence having at least 80%, 81%, 82%, 83%, 84%, preferably 85%, 86%, 87%, 88%, 89%, 90%, 91%, 92%, 93%, 94%, 95%, 96%, 97%, 98%, 99% or 100% sequence identity to SEQ ID NO: 40, and
wherein the LCDR1, 2, and 3 comprised in the VL domain consist of SEQ ID NO: 33, 34 and 35, respectively, and wherein the HCDR1, 2 and 3 comprised in said VH domain consists of SEQ ID NO: 36, 37 and 38, respectively.

Preferably, the IL13Rα2 targeting moiety comprised in the extracellular domain is a scFv comprising a VL domain and VH domain, wherein the VL domain comprises SEQ ID NO: 39 and the VH domain comprises SEQ ID NO: 40. Preferably, the IL13Rα2 targeting moiety comprised in the extracellular domain is a scFv comprising a VL domain and VH domain, wherein the VL domain consists of SEQ ID NO: 39 and the VH domain consists of SEQ ID NO: 40.

Preferably, the IL13Rα2 targeting moiety comprised in the extracellular domain is a scFv comprising a VL domain and VH domain, wherein the VL domain comprises SEQ ID NO: 41 and the VH domain comprises SEQ ID NO: 42. Preferably, the IL13Rα2 targeting moiety comprised in the extracellular domain is a scFv comprising a VL domain and VH domain, wherein the VL domain consists of SEQ ID NO: 41 and the VH domain consists of SEQ ID NO: 42.

Most preferably, the IL13Rα2 targeting moiety comprised in the extracellular domain is a scFv that comprises, consists, or consists essentially of SEQ ID NO: 45, or a sequence that has 90%, 91%, 92%, 93%, 94%, 95%, 96%, 97%, 98%, or 100% sequence identity to SEQ ID NO: 45. Most preferably, the IL13Rα2 targeting moiety is a scFv that consists of SEQ ID NO: 45. Most preferably, the IL13Rα2 targeting moiety comprised in the extracellular domain is a scFv that comprises, consists, or consists essentially of SEQ ID NO: 47, or a sequence that has 90%, 91%, 92%, 93%, 94%, 95%, 96%, 97%, 98%, or 100% sequence identity to SEQ ID NO: 47. Most preferably, the IL13Rα2 targeting moiety is a scFv that consists of SEQ ID NO: 47.

Preferably, the IL13Rα2 targeting moiety comprised in the extracellular domain is the targeting moiety as defined in the fourth aspect or any of its embodiments (IL13Rα2_82B). Preferably, the IL13Rα2 targeting moiety comprised in the extracellular domain is a scFv comprising a VL domain and VH domain, wherein said VL domain comprises LCDR1, LCDR2 and LCDR3 polypeptides and said VH domain comprises HCDR1, HCDR2 and HCDR3 polypeptides, and wherein said VL domain comprises LCDR1, LCDR2 and LCDR3 polypeptides and said VH domain comprises HCDR1, HCDR2 and HCDR3 polypeptides, and LCDR1 consists of SEQ ID NO: 49, LCDR2 consists of SEQ ID NO: 50, LCDR3 consists of SEQ ID NO: 51, HCDR1 consists of SEQ ID NO: 52, HCDR2 consists of SEQ ID NO: 53, and HCDR3 consists of SEQ ID NO: 54.

Preferably, the IL13Rα2 targeting moiety comprised in the extracellular domain is a scFv capable of binding to human IL13Rα2 and that comprises a VL and a VH domain, wherein said VL domain comprises LCDR1, LCDR2 and LCDR3 polypeptides and said VH domain comprises HCDR1, HCDR2 and HCDR3 polypeptides, and wherein:
- the VL domain comprises a sequence having at least 80%, 81%, 82%, 83%, 84%, preferably 85%, 86%, 87%, 88%, 89%, 90%, 91%, 92%, 93%, 94%, 95%, 96%, 97%, 98%, 99% or 100% sequence identity to SEQ ID NO: 55, and
- the VH domain comprising a sequence having at least 80%, 81%, 82%, 83%, 84%, preferably 85%, 86%, 87%, 88%, 89%, 90%, 91%, 92%, 93%, 94%, 95%, 96%, 97%, 98%, 99% or 100% sequence identity to SEQ ID NO: 56, and
wherein the LCDR1, 2, and 3 comprised in the VL domain consist of SEQ ID NO: 49, 50 and 51, respectively, and wherein the HCDR1, 2 and 3 comprised in said VH domain consists of SEQ ID NO: 52, 53 and 54, respectively.

Preferably, the IL13Rα2 targeting moiety comprised in the extracellular domain is a scFv comprising a VL domain and VH domain, wherein the VL domain comprises SEQ ID NO: 55 and the VH domain comprises SEQ ID NO: 56. Preferably, the IL13Rα2 targeting moiety comprised in the extracellular domain is a scFv comprising a VL domain and VH domain, wherein the VL domain consists of SEQ ID NO: 55 and the VH domain consists of SEQ ID NO: 56.

Preferably, the IL13Rα2 targeting moiety comprised in the extracellular domain is a scFv comprising a VL domain and VH domain, wherein the VL domain comprises SEQ ID NO: 57 and the VH domain comprises SEQ ID NO: 58. Preferably, the IL13Rα2 targeting moiety comprised in the extracellular domain is a scFv comprising a VL domain and VH domain, wherein the VL domain consists of SEQ ID NO: 57 and the VH domain consists of SEQ ID NO: 58.

Most preferably, the IL13Rα2 targeting moiety comprised in the extracellular domain is a scFv that comprises, consists, or consists essentially of SEQ ID NO: 61, or a sequence that has 90%, 91%, 92%, 93%, 94%, 95%, 96%, 97%, 98%, or 100% sequence identity to SEQ ID NO: 61. Most preferably, the IL13Rα2 targeting moiety is a scFV that consists of SEQ ID NO: 61. Most preferably, the IL13Rα2 targeting moiety comprised in the extracellular domain is a scFv that comprises, consists, or consists essentially of SEQ ID NO: 63, or a sequence that has 90%, 91%, 92%, 93%, 94%, 95%, 96%, 97%, 98%, or 100% sequence identity to SEQ ID NO: 63. Most preferably, the IL13Rα2 targeting moiety is a scFv that consists of SEQ ID NO: 63.

In a preferred embodiment, the VL and the VH domains comprised in the scFv comprised in the extracellular domain are linked by a peptide linker. Preferably, the peptide linker comprises, consists, or consists essentially of SEQ ID NO: 65, or a sequence that has 90%, 91%, 92%, 93%, 94%, 95%, 96%, 97%, 98%, or 100% sequence identity to SEQ ID NO: 65.

In a preferred embodiment, the scFv comprised in the extracellular domain further comprises a signal peptide that is placed at the N-terminal region of the IL13Rα2 targeting moiety, preferably of the ScFv. Preferably, the signal peptide comprises, consists, or consists essentially of SEQ ID NO: 66, or a sequence that has 90%, 91%, 92%, 93%, 94%, 95%, 96%, 97%, 98%, or 100% sequence identity to SEQ ID NO: 66.

### b) transmembrane domain

The transmembrane domain may be derived either from a natural or a synthetic source. When the source is natural, the domain may be derived from any membrane-bound or transmembrane protein. Transmembrane regions may comprise at least the transmembrane region(s) of the α-, β- or ζ- chain of CD28, CD3, CD45, CD4, CD8, CD9, CD16, CD22, CD33, CD37, CD64, CD80, CD86, CD134, CD137, or CD154.

A transmembrane domain may be synthetic or a variant of a naturally occurring transmembrane domain. In some embodiments, synthetic or variant transmembrane domains comprise predominantly hydrophobic residues such as leucine and valine.

In some embodiments, the transmembrane domain comprises the transmembrane domain of CD28, CD3, CD45, CD4, CD8, CD9, CD16, CD22, CD33, CD37, CD64, CD80, CD86, CD134, CD137, CD154, or a variant thereof, wherein the variant thereof has a 95% sequence identity.

In some embodiments, the transmembrane domain comprises the transmembrane domain of CD28, CD3, CD45, CD4, CD8, CD9, CD16, CD22, CD33, CD37, CD64, CD80, CD86, CD134, CD137, CD154, or a variant thereof, wherein the variant thereof has a 98% sequence identity.

In some embodiments, the transmembrane domain comprises the transmembrane domain of CD28, CD3, CD45, CD4, CD8, CD9, CD16, CD22, CD33, CD37, CD64, CD80, CD86, CD134, CD137, or CD154.

In some embodiments, the transmembrane domain comprises the transmembrane domain of CD8 or a variant thereof, wherein the variant thereof has a 95% sequence identity.

In some embodiments, the transmembrane domain comprises the transmembrane domain of CD8 or a variant thereof, wherein the variant thereof has a 98% sequence identity.

In some embodiments, the transmembrane domain comprises the transmembrane domain of CD8.

In some embodiments, the transmembrane domain comprises SEQ ID NO: 67 or a sequence that has 95% sequence identity to SEQ ID NO: 67. In some embodiments, the transmembrane domain comprises SEQ ID NO: 67 or a sequence that has 98% sequence identity to SEQ ID NO: 67. In some embodiments, the transmembrane domain comprises SEQ ID NO: 67. In some embodiments, the transmembrane domain consists of SEQ ID NO: 67.

### c) Intracellular signaling domain

The intracellular signaling domain provides for the activation of at least one function of the cell expressing the CAR upon binding to the ligand expressed on tumor cells. In some embodiments, the intracellular signaling domain contains one or more intracellular signaling domains. In some embodiments, the intracellular signaling domain is a portion of and/or a variant of an intracellular signaling domain that provides for activation of at least one function of the CAR-comprising cell.

In some embodiments, the intracellular signaling domain comprises the intracellular domain of CD3ζ, FcRy, CD3γ, CD3δ, CD3ε, CD5, CD22, CD79a, CD79b, CD66b, or a variant thereof, wherein the variant thereof has a 95% sequence identity.

In some embodiments, the intracellular signaling domain comprises the intracellular domain of CD3ζ, FcRy, CD3γ, CD3δ, CD3ε, CD5, CD22, CD79a, CD79b, CD66b, or a variant thereof, wherein the variant thereof has a 98% sequence identity.

In some embodiments, the intracellular signaling domain comprises the intracellular domain of CD3ζ, FcRy, CD3γ, CD3δ, CD3ε, CD5, CD22, CD79a, CD79b or CD66b.

In some embodiments, the intracellular signaling domain comprises the intracellular domain of CD3ζ or a variant thereof, wherein the variant thereof has a 95% sequence identity.

In some embodiments, the intracellular signaling domain comprises the intracellular domain of CD3ζ or a variant thereof, wherein the variant thereof has a 98% sequence identity.

In some embodiments, the intracellular signaling domain comprises the intracellular domain of CD3ζ.

In some embodiments, the intracellular signaling domain comprises SEQ ID NO: 68 or a sequence that has 95% sequence identity to SEQ ID NO: 68. In some embodiments, the intracellular signaling domain comprises SEQ ID NO: 68 or a sequence that has 98% sequence identity to SEQ ID NO: 68. In some embodiments, the intracellular signaling domain comprises SEQ ID NO: 68 or a sequence that has 99% sequence identity to SEQ ID NO: 68. In some embodiments, the intracellular signaling domain comprises SEQ ID NO: 68. In some embodiments, the intracellular signaling domain consists of SEQ ID NO: 68.

Optionally, at least a costimulatory signaling domain may also be present in the CAR according to the first aspect or any of its embodiments:

### d) Costimulatory signaling domain

In some embodiments, the CAR may further comprise a costimulatory signaling domain.

In some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, CD276 or a variant thereof, wherein the variant thereof has a 95% sequence identity.

In some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, CD276 or a variant thereof, wherein the variant thereof has a 98% sequence identity.

In some embodiments, the costimulatory signaling domain comprises the intracellular domain of CD27, CD28, CD137, CD134, CD30, CD40, lymphocyte function-associated antigen-1 (LFA-1), CD2, CD7, LIGHT, NKG2C, or CD276.

In some embodiments, the costimulatory signaling domain comprises the intracellular domain of CD137 or a variant thereof, wherein the variant thereof has a 95% sequence identity.

In some embodiments, the costimulatory signaling domain comprises the intracellular domain of CD137 or a variant thereof, wherein the variant thereof has a 98% sequence identity.

In some embodiments, the costimulatory signaling domain comprises the intracellular domain of CD137. In some embodiments, the costimulatory signaling domain comprises SEQ ID NO: 69 or a sequence that has 95% sequence identity to SEQ ID NO: 69. In some embodiments, the costimulatory signaling domain comprises SEQ ID NO: 69 or a sequence that has 98% sequence identity to SEQ ID NO: 69. In some embodiments, the costimulatory signaling domain comprises SEQ ID NO: 69 or a sequence that has 99% sequence identity to SEQ ID NO: 69. In some embodiments, the costimulatory signaling domain comprises SEQ ID NO: 69. In some embodiments, the costimulatory signaling domain consists of SEQ ID NO: 69.

### Full sequence CARs according to the first aspect of the present invention

In some embodiments, the CAR comprises:
a) an extracellular domain comprising a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 1, LCDR2 consists of SEQ ID NO: 2, LCDR3 consists of SEQ ID NO: 3, HCDR1 consists of SEQ ID NO: 4, HCDR2 consists of SEQ ID NO: 5, and HCDR3 consists of SEQ ID NO: 6;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain comprising a scFv comprising a VL and a VH domain, wherein the VL domain comprises a sequence with at least 80% sequence identity to SEQ ID NO: 7, and wherein said VL domain comprises CDR polypeptides of LCDR1 according to SEQ ID NO: 1, LCDR2 according to SEQ ID NO: 2, and LCDR3 according to SEQ ID NO: 3, and said VH domain comprises a sequence with at least 80% sequence identity to SEQ ID NO: 8, and wherein said VH domain comprises CDR polypeptides of HCDR1 according to SEQ ID NO: 4, HCDR2 according to SEQ ID NO: 5, and HCDR3 according to SEQ ID NO: 6;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain comprises SEQ ID NO: 7 and said VH domain comprises SEQ ID NO: 8, or
   - said VL domain comprises SEQ ID NO: 9 and said VH domain comprises SEQ ID NO: 10;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain consists of SEQ ID NO: 7 and said VH domain comprises SEQ ID NO: 8, or
   - said VL domain consists of SEQ ID NO: 9 and said VH domain comprises SEQ ID NO: 10,
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain consists of SEQ ID NO: 7 and said VH domain comprises SEQ ID NO: 8, or
   - said VL domain consists of SEQ ID NO: 9 and said VH domain comprises SEQ ID NO: 10,
b) a transmembrane domain comprising SEQ ID NO: 67;
c) a signaling domain comprising SEQ ID NO: 68; and
d) a costimulatory signaling domain comprising SEQ ID NO: 69.

In some embodiments, the CAR comprises:
a) an extracellular domain that is a scFv comprising SEQ ID NO: 13 or 15, preferably SEQ ID NO: 13;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv consisting of SEQ ID NO: 13 or 15, preferably SEQ ID NO: 13;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises SEQ ID NO: 14, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 14. Preferably, the CAR comprises or consists of SEQ ID NO: 14. In some embodiments, the CAR comprises SEQ ID NO: 16, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 16. Preferably, the CAR comprises or consists of SEQ ID NO: 16.

In some embodiments, the CAR comprises:
a) an extracellular domain comprising a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 17, LCDR2 consists of SEQ ID NO: 18, LCDR3 consists of SEQ ID NO: 19, HCDR1 consists of SEQ ID NO: 20, HCDR2 consists of SEQ ID NO: 21, and HCDR3 consists of SEQ ID NO: 22;
b) a transmembrane domain comprising the transmembrane domain of CD8;
c) signaling domain comprising the intracellular domain of CD3ζ; and
d) a costimulatory signaling domain comprising the intracellular domain of CD137.

In some embodiments, the CAR comprises:
a) an extracellular domain comprising a scFv comprising a VL and a VH domain, wherein the VL domain comprises a sequence with at least 80% sequence identity to SEQ ID NO: 23, and wherein said VL domain comprises CDR polypeptides of LCDR1 according to SEQ ID NO: 17, LCDR2 according to SEQ ID NO: 18, and LCDR3 according to SEQ ID NO: 19, and said VH domain comprises a sequence with at least 80% sequence identity to SEQ ID NO: 24, and wherein said VH domain comprises CDR polypeptides of HCDR1 according to SEQ ID NO: 20, HCDR2 according to SEQ ID NO: 21, and HCDR3 according to SEQ ID NO: 22;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain comprises SEQ ID NO: 23 and said VH domain comprises SEQ ID NO: 24, or
   - said VL domain comprises SEQ ID NO: 25 and said VH domain comprises SEQ ID NO: 26;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain consists of SEQ ID NO: 23 and said VH domain comprises SEQ ID NO: 24, or
   - said VL domain consists of SEQ ID NO: 25 and said VH domain comprises SEQ ID NO: 26,
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain consists of SEQ ID NO: 23 and said VH domain comprises SEQ ID NO: 24, or
   - said VL domain consists of SEQ ID NO: 25 and said VH domain comprises SEQ ID NO: 26,
b) a transmembrane domain comprising SEQ ID NO: 67;
c) a signaling domain comprising SEQ ID NO: 68; and
d) a costimulatory signaling domain comprising SEQ ID NO: 69.

In some embodiments, the CAR comprises:
a) an extracellular domain that is a scFv comprising SEQ ID NO: 29 or 31, preferably SEQ ID NO: 29;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv consisting of SEQ ID NO: 29 or 31, preferably SEQ ID NO: 29;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises SEQ ID NO: 30, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 30. Preferably, the CAR comprises or consists of SEQ ID NO: 30. In some embodiments, the CAR comprises SEQ ID NO: 32 or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 32. Preferably, the CAR comprises or consists of SEQ ID NO: 32.

In some embodiments, the CAR comprises:
a) an extracellular domain comprising a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 33, LCDR2 consists of SEQ ID NO: 34, LCDR3 consists of SEQ ID NO: 35, HCDR1 consists of SEQ ID NO: 36, HCDR2 consists of SEQ ID NO: 37, and HCDR3 consists of SEQ ID NO: 38;
b) a transmembrane domain comprising the transmembrane domain of CD8;
c) signaling domain comprising the intracellular domain of CD3ζ; and
d) a costimulatory signaling domain comprising the intracellular domain of CD137.

In some embodiments, the CAR comprises:
a) an extracellular domain comprising a scFv comprising a VL and a VH domain, wherein the VL domain comprises a sequence with at least 80% sequence identity to SEQ ID NO: 39, and wherein said VL domain comprises CDR polypeptides of LCDR1 according to SEQ ID NO: 33, LCDR2 according to SEQ ID NO: 34, and LCDR3 according to SEQ ID NO: 35, and said VH domain comprises a sequence with at least 80% sequence identity to SEQ ID NO: 40, and wherein said VH domain comprises CDR polypeptides of HCDR1 according to SEQ ID NO: 36, HCDR2 according to SEQ ID NO: 37, and HCDR3 according to SEQ ID NO: 38;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain comprises SEQ ID NO: 39 and said VH domain comprises SEQ ID NO: 40, or
   - said VL domain comprises SEQ ID NO: 41 and said VH domain comprises SEQ ID NO: 42;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain consists of SEQ ID NO: 39 and said VH domain comprises SEQ ID NO: 40, or
   - said VL domain consists of SEQ ID NO: 41 and said VH domain comprises SEQ ID NO: 42,
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain consists of SEQ ID NO: 39 and said VH domain comprises SEQ ID NO: 40, or
   - said VL domain consists of SEQ ID NO: 41 and said VH domain comprises SEQ ID NO: 42,
b) a transmembrane domain comprising SEQ ID NO: 67;
c) a signaling domain comprising SEQ ID NO: 68; and
d) a costimulatory signaling domain comprising SEQ ID NO: 69.

In some embodiments, the CAR comprises:
a) an extracellular domain that is a scFv comprising SEQ ID NO: 45 or 47, preferably SEQ ID NO: 45;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFV consisting of SEQ ID NO: 45 or 47, preferably SEQ ID NO: 45;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises SEQ ID NO: 46, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 46. Preferably, the CAR comprises or consists of SEQ ID NO: 46. In some embodiments, the CAR comprises SEQ ID NO: 48, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 48. Preferably, the CAR comprises or consists of SEQ ID NO: 48.

In some embodiments, the CAR comprises:
a) an extracellular domain comprising a scFv comprising a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 49, LCDR2 consists of SEQ ID NO: 50, LCDR3 consists of SEQ ID NO: 51, HCDR1 consists of SEQ ID NO: 52, HCDR2 consists of SEQ ID NO: 53, and HCDR3 consists of SEQ ID NO: 54;
b) a transmembrane domain comprising the transmembrane domain of CD8;
c) signaling domain comprising the intracellular domain of CD3ζ; and
d) a costimulatory signaling domain comprising the intracellular domain of CD137.

In some embodiments, the CAR comprises:
a) an extracellular domain comprising a scFv comprising a VL and a VH domain, wherein the VL domain comprises a sequence with at least 80% sequence identity to SEQ ID NO: 55, and wherein said VL domain comprises CDR polypeptides of LCDR1 according to SEQ ID NO: 49, LCDR2 according to SEQ ID NO: 50, and LCDR3 according to SEQ ID NO: 51, and said VH domain comprises a sequence with at least 80% sequence identity to SEQ ID NO: 56, and wherein said VH domain comprises CDR polypeptides of HCDR1 according to SEQ ID NO: 52, HCDR2 according to SEQ ID NO: 53, and HCDR3 according to SEQ ID NO: 54;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain comprises SEQ ID NO: 55 and said VH domain comprises SEQ ID NO: 56, or
   - said VL domain comprises SEQ ID NO: 57 and said VH domain comprises SEQ ID NO: 58;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain consists of SEQ ID NO: 55 and said VH domain comprises SEQ ID NO: 56, or
   - said VL domain consists of SEQ ID NO: 57 and said VH domain comprises SEQ ID NO: 58;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv comprising a VL and a VH domain, wherein:
   - said VL domain consists of SEQ ID NO: 55 and said VH domain comprises SEQ ID NO: 56, or
   - said VL domain consists of SEQ ID NO: 57 and said VH domain comprises SEQ ID NO: 58;
b) a transmembrane domain comprising SEQ ID NO: 67;
c) a signaling domain comprising SEQ ID NO: 68; and
d) a costimulatory signaling domain comprising SEQ ID NO: 69.

In some embodiments, the CAR comprises:
a) an extracellular domain that is a scFv comprising SEQ ID NO: 61 or 63, preferably SEQ ID NO: 61;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises:
a) an extracellular domain that is a scFv consisting of SEQ ID NO: 61 or 63, preferably SEQ ID NO: 61;
b) a transmembrane domain comprising the transmembrane domain of CD8;
c) a signaling domain comprising the intracellular domain of CD3ζ; and
d) a costimulatory signaling domain comprising the intracellular domain of CD137.

In some embodiments, the CAR comprises SEQ ID NO: 62, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 62. Preferably, the CAR comprises or consists of SEQ ID NO: 62. In some embodiments, the CAR comprises SEQ ID NO: 64, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 64. Preferably, the CAR comprises or consists of SEQ ID NO: 64.

### Nucleic acid of the invention

In a **fifth aspect,** the present invention provides a nucleic acid encoding any one of the IL13Rα2 targeting moieties of the present invention (disclosed in the first, second, third, and fourth aspects, including any one of the CARs disclosed above). The nucleic acid sequence that encodes the chimeric receptor links together a number of modular components that can be excised and replaced with other components in order to customize the chimeric receptor for efficient T cell activation and recognition of IL13Rα2.

In some embodiments, the nucleic acid is suitable for transducing or transforming a cell. In some embodiments, the nucleic acid is suitable for transducing or transforming a T cell for use in adoptive immunotherapy.

In some embodiments, the nucleic acid is codon optimized for expression in mammalian cells. Codon optimization methods are known in the art.

The nucleic acid of the present invention may be comprised in a γ-retroviral or lentiviral vector which can be used to transduce or transform a T cell. Preferably, the lentiviral vector is infective but not replicative. Preferably, the lentiviral vector lacks the sequences required for the formation of replication competent lentiviruses.

The nucleic acid may also be inserted into a cell through the use of DNA transposons, RNA transfection or genome editing techniques such as TALEN, ZFN and CRISPR/Cas9.

In some embodiments, the nucleic acid comprises SEQ ID NO: 11 and 12, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 11 and 12, respectively. Preferably, the CAR comprises or consists of SEQ ID NO: 11 and 12.

In some embodiments, the nucleic acid comprises SEQ ID NO: 27 and 28, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 27 and 28, respectively. Preferably, the CAR comprises or consists of SEQ ID NO: 27 and 28.

In some embodiments, the nucleic acid comprises SEQ ID NO: 43 and 44, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 43 and 44, respectively. Preferably, the CAR comprises or consists of SEQ ID NO: 43 and 44.

In some embodiments, the nucleic acid comprises SEQ ID NO: 59 and 60, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% sequence identity to SEQ ID NO: 59 and 60, respectively. Preferably, the CAR comprises or consists of SEQ ID NO: 59 and 60.

### Cells of the invention

In a **sixth aspect,** the present invention provides a cell comprising the nucleic acid of the invention and/or the CARs of the invention. In some embodiments, the cell is a T cell (referred to as a CART).

In some embodiments, the cell is a naive T cell, memory stem T cell or central memory T cell. It is currently thought that these cells are better suited for adaptive immunotherapy.

In some embodiments, the cell is an autologous T cell. The term "autologous cell" refers to a cell obtained from the same patient that is to be treated using any one of the methods of the present invention.

In some embodiments, the cell is an allo-tolerant T cell. The term "all-tolerant cell" refers to a cell that has been engineered to decrease the risk of a Graft-versus-host disease response. In some embodiments, this is achieved by genomic editing-mediated deletion of TCR and/or β2-microglobulin. Allo-tolerant cells are known in the art.

In some embodiments, the T cell is a human cell, preferably autologous human cell. In some embodiments, the cell is a lymphoid precursor, embryonic stem cell or an induced pluripotent stem cell with the capacity to differentiate into a mature T cell.

In some embodiments, the T cells expresses or comprises the scFv of the IL13Rα2_6A defined in the first aspect or any of its embodiments, preferably comprising or consisting of SEQ ID NOs: 13 or 15, preferably SEQ ID NO: 13. In some embodiments, the T cells expresses or comprises the CAR IL13Rα2_6A defined in the first aspect or any of its embodiments, preferably comprising or consisting of SEQ ID NOs: 14 or 16, preferably SEQ ID NO: 14.

In some embodiments, the T cells expresses or comprises the scFv of the IL13Rα2_46A defined in the second aspect or any of its embodiments, preferably comprising or consisting of SEQ ID NOs: 29 or 31, preferably SEQ ID NO: 29. In some embodiments, the T cells expresses or comprises the CAR IL13Rα2_46A defined in the second aspect or any of its embodiments, preferably comprising or consisting of SEQ ID NOs: 30 or 32, preferably SEQ ID NO: 30.

In some embodiments, the T cells expresses or comprises the scFv of the IL13Rα2_143A defined in the third aspect or any of its embodiments, preferably comprising or consisting of SEQ ID NOs: 45 or 47, preferably SEQ ID NO: 45. In some embodiments, the T cells expresses or comprises the CAR IL13Rα2_143A defined in the third aspect or any of its embodiments, preferably comprising or consisting of SEQ ID NOs: 46 or 48, preferably SEQ ID NO: 46.

In some embodiments, the T cells expresses or comprises the scFv of the IL13Rα2_82B defined in the fourth aspect or any of its embodiments, preferably comprising or consisting of SEQ ID NOs: 61 or 63, preferably SEQ ID NO: 63. In some embodiments, the T cells expresses or comprises the CAR IL13Rα2_82B defined in the fourth aspect or any of its embodiments, preferably comprising or consisting of SEQ ID NOs: 62 or 64, preferably SEQ ID NO: 62.

### Pharmaceutical composition of the invention

In a **seventh aspect,** the present invention provides a pharmaceutical composition comprising the nucleic acid of the invention and/or a plurality of cells of the present invention, and a pharmaceutically acceptable carrier or diluent.

A pharmaceutical composition as described herein may also contain other substances. These substances include, but are not limited to, cryoprotectants, surfactants, antioxidants, and stabilizing agents. The term "cryoprotectant" as used herein, includes agents which provide stability to the CARTs against freezing-induced stresses. Nonlimiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used. In an embodiment, the pharmaceutical composition comprises albumin, preferably human albumin, as stabilizer.

In some embodiments, the cells are formulated by first harvesting them from their culture medium, and then washing and concentrating the cells in a medium and container system suitable for administration (a "pharmaceutically acceptable" carrier) in a therapeutically effective amount. Suitable infusion medium can be any isotonic medium formulation, typically normal saline, Normosol R (Abbott) or Plasma-Lyte A (Baxter), but also 5% dextrose in water or Ringer's lactate can be utilized. The infusion medium can be supplemented with human serum albumin, fetal bovine serum, or other human serum components.

### Methods of treatment of the invention

In an **eighth aspect,** the present invention provides uses and/or method of treating a disease comprising administering the IL13Rα2 targeting moieties of the invention (disclosed in the first, second, third, and fourth aspects, including any one of the CARs disclosed above), the nucleic acid of the invention, cell of the invention or the pharmaceutical composition of the invention to a patient in need thereof. Thus, these aspects refer to the use of the IL13Rα2 targeting moieties of the invention (disclosed in the first, second, third, and fourth aspects, including any one of the CARs disclosed above), the nucleic acid of the invention, cell of the invention or the pharmaceutical composition of the invention in therapy or to manufacture a medicament.

The use according to the present invention may be to target malignant cells that overexpress IL13Rα2. Hence, the use involves a method of treating or preventing or ameliorating a IL13Rα2-positive cancer comprising administering the IL13Rα2 targeting moieties of the invention (disclosed in the first, second, third, and fourth aspects, including any one of the CARs disclosed above), the nucleic acid of the invention, cell of the invention or the pharmaceutical composition of the invention to a patient in need thereof. Preferably, the IL13Rα2-positive cancer is selected from the list consisting of glioblastoma, diffuse intrinsic pontine glioma (DIPG), pancreatic cancer, ovarian cancer, breast cancer, colon cancer and melanoma. Preferably, the IL13Rα2-positive cancer is glioblastoma or DIPG.

As shown in the Examples, the IL13Rα2 targeting moieties developed in the present invention are not only effective in reducing the tumour but also in avoiding tumor development before it appears. Hence, the methods and uses disclosed herein include the therapeutic and prophylactic treatment of patient in need thereof. Preferably, the uses disclosed herein include methods for the treatment of a IL13Rα2-positive cancer, preferably glioblastoma or DIPG. Also preferably, the uses disclosed herein include methods for the prevention of a IL13Rα2-positive cancer, preferably glioblastoma or DIPG.

Preferably, the patient in need thereof is a human, most preferably a human having or suffering from a IL13Rα2 positive cancer, preferably glioblastoma or DIPG.

In an embodiment, the uses or methods of treatment comprises a first step of a) providing a population of blood cells, b) isolating T cells from those blood cells, c) modifying the T cells to express the IL13Rα2 targeting moieties preferably the CARs, of the present invention, and d) administering said modified T to a patient in need thereof. Preferably, the population of blood cells is obtained from the same patient to which the cells are administered in step d) (autologous therapy).

In some embodiments, the patient or subject in need thereof is administered a therapeutically effective amount of cells. In an embodiment, a therapeutically effective amount or effective dose may be administered 1, 2, 3, 4, 5, 6, or more than 6 times a month or a year, or during at least 1, 2, 3, 4, 5, 6, 7, 8, 9, 10, 11, 12, or more than 12 months or years. In an embodiment, a therapeutically effective amount or effective dose may be administered one, two, three, four, five, six, seven, or more than seven times during a period about 1, 2, 3, 4, 5, 6, 7, 8, 9, 10, or more than 10 years.

In an embodiment, the therapeutically effective amount or effective dose can be fractionated, independently of the number of times in which such therapeutically effective amount or effective dose is administrated to the patient, in order to reduce the likelihood of appearance of toxicity. The term "fractionated" means that the effective dose is delivered over the course of several days in several administrations, wherein each administration comprises a partial dose. This is also called herein a fractionated administration scheme.

In an embodiment, the cells are administered together with lymphodepletive chemotherapy. In an embodiment, prolonged immunosuppression is not applied to the patient.

In some embodiments, the cell or pharmaceutical composition is administered intravenously, intraperitoneally, intracavitary, intraventricular, into the bone marrow, into the lymph node, and /or into cerebrospinal fluid. Preferably, the cell or pharmaceutical composition is administered intracavitary or intraventricular.

In some embodiments, the method comprises a combination therapy. In some embodiments, the method comprises further administering an immune checkpoint. In a further embodiment, the method comprises further administering an immune checkpoint inhibitor and/or an IAP inhibitor.

In some embodiments, the cell or pharmaceutical composition as described herein is administered in combination with chemotherapeutic agents and/or immunosuppressants. In an embodiment, a patient is first treated with a chemotherapeutic agent that inhibits or destroys other immune cells followed by the cell or pharmaceutical composition described herein. In some cases, chemotherapy may be avoided entirely.

In some embodiments, the patient to be treated with the method of the present invention is in complete or near-complete remission after treatment with another therapy.

### Kits

In a **ninth aspect,** the present invention provides kits and their use according to any of the previous aspects. The materials described above as well as other materials can be packaged together in any suitable combination as a kit useful for performing, or aiding in the performance of, the disclosed method. It is useful if the kit components in a given kit are designed and adapted for use together in the disclosed method.

Each embodiment disclosed herein is contemplated as being applicable to each of the other disclosed embodiments. Thus, all combinations of the various elements described herein are within the scope of the invention.

### SEQUENCE LISTING

The invention is described below by the following examples, which must be considered as merely illustrative and in no case limiting of the scope of the present invention.

### IL13Rα2_6A:

IL13Rα2_6A VL-CDR1-SEQ ID NO: 1: KASQDIKSYLS
IL13Rα2_6A VL-CDR2- SEQ ID NO: 2: RANRLVD
IL13Rα2_6A VL-CDR3- SEQ ID NO: 3: LQYDEFPLT
IL13Rα2_6A VH-CDR1 - SEQ ID NO: 4: EYTMH
IL13Rα2_6A VH-CDR2- SEQ ID NO: 5: IINPNNGGTNYNQKFKG
IL13Rα2_6A VH-CDR3- SEQ ID NO: 6: PSATHYFDY
IL13Rα2 6A VL murine amino acid sequence - SEQ ID NO: 7 
DIKMTQSPSSMYASLGERVTITCKASQDIKSYLSWFQQKPGKSPKTLIYRANRLVDG
VPSRFSGSGSGQDYSLTISSLEYEDMGIYYCLQYDEFPLTFGAGTKLELK
IL13Rα2 6A VH murine amino acid sequence - SEQ ID NO: 8 
EVQLQQSGPELVKPGASVKISCKTSGYTFTEYTMHWVKQSHGKSLEWIGIINPNNGG
TNYNQKFKGKATLTVDKSSSTAYMELRSLTSEDSAVYYCARPSATHYFDYWGQGTTL
TVSS
IL13Rα2 6A VL humanized amino acid sequence - SEQ ID NO: 9 
DIQMTQSPSSLYASVGDRVTITCKASQDIKSYLSWYQQKPGKAPKTLIYRANRLVDG
VPSRFSGSGSGQDYTLTISSLQYEDMGIYYCLQYDEFPLTFGAGTKVEIK
IL13Rα2 6A VH humanized amino acid sequence - SEQ ID NO: 10 
QVQLQQSGPEVVKPGASVKMSCKTSGYTFTEYTMHWVKQTHGRSLEWIGIINPNNG
GTNYNQKFKGRATMTVDKSSSTAYMQLRSLTSDDTAVYYCARPSATHYFDYWGQGT
TVTVSS
IL13Rα2 6A VH murine nucleotide sequence - SEQ ID NO: 11 
GAAGTCCAGCTTCAACAGTCTGGGCCTGAATTGGTGAAACCCGGCGCGTCCGTTA
AAATCAGCTGCAAGACCTCTGGATACACATTTACCGAGTATACAATGCATTGGGTA
AAGCAGAGCCACGGTAAAAGTCTGGAATGGATCGGCATTATCAATCCAAATAACG
GGGGCACTAATTATAACCAGAAATTCAAAGGGAAAGCTACACTCACCGTGGATAA
GTCATCCTCGACGGCATATATGGAACTGCGGTCCCTTACCTCAGAGGATTCAGCA
GTCTACTATTGTGCCCGTCCAAGCGCCACACACTATTTTGACTACTGGGGACAAG
GAACCACACTAACTGTGTCTTCG
IL13Rα2 6A VL murine nucleotide sequence - SEQ ID NO: 12 
GACATAAAGATGACCCAGTCACCGTCTAGCATGTATGCCAGTCTGGGAGAGCGAG
TGACTATCACATGCAAGGCCAGCCAGGACATAAAGTCCTACCTCAGTTGGTTCCA
ACAGAAGCCTGGCAAGAGTCCCAAAACTCTGATTTACAGGGCTAACAGACTGGTT
GACGGGGTGCCCTCCCGCTTCTCTGGCAGCGGAAGCGGACAGGATTACTCCCTG
ACGATCTCCTCACTCGAGTACGAGGATATGGGCATTTACTACTGTTTGCAGTATGA
CGAGTTTCCTTTAACTTTCGGAGCTGGCACCAAGCTCGAACTGAAG
IL13Rα2 6A murine scFv amino acid sequence- SEQ ID NO: 13 
MALPVTGLLLSLGLLLHAARPTGDIKMTQSPSSMYASLGERVTITCKASQDIKSYLSWF
QQKPGKSPKTLIYRANRLVDGVPSRFSGSGSGQDYSLTISSLEYEDMGIYYCLQYDEF
PLTFGAGTKLELKGGGGSGGGGSGGGGSGGGGSEVQLQQSGPELVKPGASVKISC
KTSGYTFTEYTMHWVKQSHGKSLEWIGIINPNNGGTNYNQKFKGKATLTVDKSSSTA
YMELRSLTSEDSAVYYCARPSATHYFDYWGQGTTLTVSS
IL13Rα2 6A murine CAR amino acid sequence - SEQ ID NO: 14 
DIKMTQSPSSMYASLGERVTITCKASQDIKSYLSWFQQKPGKSPKTLIYRANRLVDGV
PSRFSGSGSGQDYSLTISSLEYEDMGIYYCLQYDEFPLTFGAGTKLELKGGGGSGGG
GSGGGGSGGGGSEVQLQQSGPELVKPGASVKISCKTSGYTFTEYTMHWVKQSHGK
SLEWIGIINPNNGGTNYNQKFKGKATLTVDKSSSTAYMELRSLTSEDSAVYYCARPSA
THYFDYWGQGTTLTVSSTTTPAPRPPTPAPTIASQPLSLRPEACRPAAGGAVHTRGL
DFACDIYIWAPLAGTCGVLLLSLVITLYCKRGRKKLLYIFKQPFMRPVQTTQEEDGCSC
 
RFPEEEEGGCELRVKFSRSADAPAYQQGQNQLYNELNLGRREEYDVLDKRRGRDPE
MGGKPQRRKNPQEGLYNELQKDKMAEAYSEIGMKGERRRGKGHDGLYQGLSTATK
DTYDALHMQALPPR
IL13Rα2 6A humanized scFv amino acid sequence- SEQ ID NO: 15 
MALPVTGLLLSLGLLLHAARPTGDIQMTQSPSSLYASVGDRVTITCKASQDIKSYLSWY
QQKPGKAPKTLIYRANRLVDGVPSRFSGSGSGQDYTLTISSLQYEDMGIYYCLQYDEF
PLTFGAGTKVEIKGGGGSGGGGSGGGGSGGGGSQVQLQQSGPEVVKPGASVKMS
CKTSGYTFTEYTMHWVKQTHGRSLEWIGIINPNNGGTNYNQKFKGRATMTVDKSSST
AYMQLRSLTSDDTAVYYCARPSATHYFDYWGQGTTVTVSS
IL13Rα2 6A humanized CAR amino acid sequence- SEQ ID NO: 16

DIQMTQSPSSLYASVGDRVTITCKASQDIKSYLSWYQQKPGKAPKTLIYRANRLVDGV
PSRFSGSGSGQDYTLTISSLQYEDMGIYYCLQYDEFPLTFGAGTKVEIKGGGGSGGG
GSGGGGSGGGGSQVQLQQSGPEVVKPGASVKMSCKTSGYTFTEYTMHWVKQTHG
RSLEWIGIINPNNGGTNYNQKFKGRATMTVDKSSSTAYMQLRSLTSDDTAVYYCARP
SATHYFDYWGQGTTVTVSSTTTPAPRPPTPAPTIASQPLSLRPEACRPAAGGAVHTR
GLDFACDIYIWAPLAGTCGVLLLSLVITLYCKRGRKKLLYIFKQPFMRPVQTTQEEDGC
SCRFPEEEEGGCELRVKFSRSADAPAYQQGQNQLYNELNLGRREEYDVLDKRRGRD
PEMGGKPQRRKNPQEGLYNELQKDKMAEAYSEIGMKGERRRGKGHDGLYQGLSTA
TKDTYDALHMQALPPR

### IL13Rα2_46A:

IL13Rα2_46A VL-CDR1- SEQ ID NO: 17: TASSSVTSSYLH
IL13Rα2_46A VL-CDR2- SEQ ID NO: 18: STSNLAS
IL13Rα2_46A VL-CDR3-SEQ ID NO: 19: HQYHRSPLT
IL13Rα2_46A VH-CDR1 - SEQ ID NO: 20: KYGVH
IL13Rα2_46A VH-CDR2-SEQ ID NO: 21: VIWSGGSTDYNAAFIS
IL13Rα2_46A VH-CDR3- SEQ ID NO: 22: DFGDTMDY
IL13Rα2 46A VL murine amino acid sequence - SEQ ID NO: 23 
QIVLTQSPAIMSASLGERVTMTCTASSSVTSSYLHWYQQKPGSSPKLWIFSTSNLAS
GVPARFSGSGSGTSYSLTISSMEAEDAATYYCHQYHRSPLTFGAGTKLELK
IL13Rα2 46A VH murine amino acid sequence - SEQ ID NO: 24 
QVQLKQSGPGLVQPSQSLSITCTVSGFSITKYGVHWVRLSPGKGLEWLGVIWSGGST
DYNAAFISRLNISKDNSKSQVFFKMNSLQANDTAIYYCARDFGDTMDYWGQGTSVTV
SS
IL13Rα2 46A VL humanized amino acid sequence - SEQ ID NO: 25 
EIVLTQSPSILSASVGEDVTITCTASSSVTSSYLHWYQQKPGSAPKLWIYSTSNLAS
GVPSRFSGSGSGTSFTLTISSLQAEDAATYYCHQYHRSPLTFGAGTKLEIK
IL13Rα2 46A VH humanized amino acid sequence - SEQ ID NO: 26 
QVQLQESGPGLVKPETSLSITCTVSGFSITKYGVHWVRLSPGKGLEWLGVIWSGGST
DYNAAFISRVNISKDNSKNQVFFKMNSLQANDTAVYYCARDFGDTMDYWGQGTTVT
VSS
IL13Rα2 46A VL murine nucleotide sequence - SEQ ID NO: 27 
CAAATCGTGCTGACGCAGAGTCCCGCCATCATGTCTGCAAGCCTGGGCGAGCGC
GTGACCATGACTTGTACCGCCAGCAGCTCTGTTACATCTTCCTACCTCCACTGGTA
TCAGCAGAAGCCAGGCTCAAGTCCCAAGCTGTGGATTTTCAGTACTTCCAACTTA
GCATCGGGTGTCCCTGCCCGATTCAGCGGGAGTGGGTCGGGAACTTCCTATTCA
TTGACCATTTCTTCCATGGAAGCGGAAGACGCCGCGACCTACTATTGCCATCAGT
ATCACAGAAGCCCGCTGACATTTGGCGCTGGAACAAAACTCGAGCTAAAG
IL13Rα2 46A VH murine nucleotide sequence - SEQ ID NO: 28 
CAGGTGCAGCTGAAGCAGTCTGGACCTGGGTTGGTACAACCCTCCCAATCACTTA
GCATCACATGCACGGTGTCAGGCTTTAGCATCACAAAGTACGGTGTGCATTGGGT
TCGGCTGTCACCAGGAAAAGGGCTCGAGTGGCTTGGCGTGATTTGGAGTGGGGG
AAGCACCGACTACAATGCAGCCTTCATAAGTAGGCTCAATATCTCAAAAGACAACT
CTAAAAGTCAGGTATTCTTTAAGATGAATTCCCTGCAAGCCAACGATACCGCTATA
TACTACTGTGCTCGTGATTTTGGCGATACTATGGACTATTGGGGGCAGGGAACCA
GCGTCACTGTCTCCTCC
IL13Rα2 46A murine scFv amino acid sequence- SEQ ID NO: 29 
MALPVTGLLLSLGLLLHAARPTGQIVLTQSPAIMSASLGERVTMTCTASSSVTSSYLH
WYQQKPGSSPKLWIFSTSNLASGVPARFSGSGSGTSYSLTISSMEAEDAATYYCHQY
HRSPLTFGAGTKLELKGGGGSGGGGSGGGGSGGGGSQVQLKQSGPGLVQPSQSL
SITCTVSGFSITKYGVHWVRLSPGKGLEWLGVIWSGGSTDYNAAFISRLNISKDNSKS
QVFFKMNSLQANDTAIYYCARDFGDTMDYWGQGTSVTVSS
IL13Rα2 46A murine CAR amino acid sequence - SEQ ID NO: 30 
QIVLTQSPAIMSASLGERVTMTCTASSSVTSSYLHWYQQKPGSSPKLWIFSTSNLASG
VPARFSGSGSGTSYSLTISSMEAEDAATYYCHQYHRSPLTFGAGTKLELKGGGGSGG
GGSGGGGSGGGGSQVQLKQSGPGLVQPSQSLSITCTVSGFSITKYGVHWVRLSPGK
GLEWLGVIWSGGSTDYNAAFISRLNISKDNSKSQVFFKMNSLQANDTAIYYCARDFGD
TMDYWGQGTSVTVSSTTTPAPRPPTPAPTIASQPLSLRPEACRPAAGGAVHTRGLDF
ACDIYIWAPLAGTCGVLLLSLVITLYCKRGRKKLLYIFKQPFMRPVQTTQEEDGCSCRF
PEEEEGGCELRVKFSRSADAPAYQQGQNQLYNELNLGRREEYDVLDKRRGRDPEM
GGKPQRRKNPQEGLYNELQKDKMAEAYSEIGMKGERRRGKGHDGLYQGLSTATKD
TYDALHMQALPPR
IL13Rα2 46A humanized scFv amino acid sequence- SEQ ID NO: 31 
MALPVTGLLLSLGLLLHAARPTGEIVLTQSPSILSASVGEDVTITCTASSSVTSSYLHWY
QQKPGSAPKLWIYSTSNLASGVPSRFSGSGSGTSFTLTISSLQAEDAATYYCHQYHR
SPLTFGAGTKLEIKGGGGSGGGGSGGGGSGGGGSQVQLQESGPGLVKPETSLSITC
TVSGFSITKYGVHWVRLSPGKGLEWLGVIWSGGSTDYNAAFISRVNISKDNSKNQVFF
KMNSLQANDTAVYYCARDFGDTMDYWGQGTTVTVSS
IL13Rα2 46A humanized CAR amino acid sequence- SEQ ID NO: 32

EIVLTQSPSILSASVGEDVTITCTASSSVTSSYLHWYQQKPGSAPKLWIYSTSNLASGV
PSRFSGSGSGTSFTLTISSLQAEDAATYYCHQYHRSPLTFGAGTKLEIKGGGGSGGG
GSGGGGSGGGGSQVQLQESGPGLVKPETSLSITCTVSGFSITKYGVHWVRLSPGKG
LEWLGVIWSGGSTDYNAAFISRVNISKDNSKNQVFFKMNSLQANDTAVYYCARDFGD
TMDYWGQGTTVTVSSTTTPAPRPPTPAPTIASQPLSLRPEACRPAAGGAVHTRGLDF
ACDIYIWAPLAGTCGVLLLSLVITLYCKRGRKKLLYIFKQPFMRPVQTTQEEDGCSCRF
PEEEEGGCELRVKFSRSADAPAYQQGQNQLYNELNLGRREEYDVLDKRRGRDPEM
GGKPQRRKNPQEGLYNELQKDKMAEAYSEIGMKGERRRGKGHDGLYQGLSTATKD
TYDALHMQALPPR

### IL13Rα2_143A:

IL13Rα2_143A VL-CDR1-SEQ ID NO: 33: KASQDINSYLS
IL13Rα2_143A VL-CDR2- SEQ ID NO: 34: RANRLVD
IL13Rα2_143A VL-CDR3-SEQ ID NO: 35: LQYDEFPLT
IL13Rα2_143A VH-CDR1 - SEQ ID NO: 36: SFGMH
IL13Rα2_143A VH-CDR2- SEQ ID NO: 37: YISSGSSTIYYADTVKG
IL13Rα2_143A VH-CDR3- SEQ ID NO: 38: KYEGYAMDY
IL13Rα2 143A VL murine amino acid sequence - SEQ ID NO: 39 
DIKMTQSPSSMYASLGERVTITCKASQDINSYLSWFQQKPGKSPKTLIYRANRLVDG
VPSRFSGSGSGQDYSLTISSLEYEDMGIYSCLQYDEFPLTFGAGTKLELK
IL13Rα2 143A VH murine amino acid sequence - SEQ ID NO: 40 
DVQLVESGGGLVQPGGSRKLSCAASGFTFSSFGMHWVRQAPEKGLEWVAYISSGSS
TIYYADTVKGRFIISRDNPKNTLFLQMTSLRSEDTAMYYCARKYEGYAMDYWGQGTS
VTVSS
IL13Rα2 143A VL humanized amino acid sequence - SEQ ID NO: 41 
DIQMTQSPSSLYASVGDRVTITCKASQDINSYLSWYQQKPGKAPKTLIYRANRLVDG
VPSRFSGSGSGQDYTLTISSLQYEDMGIYSCLQYDEFPLTFGAGTKVEIK
IL13Rα2 143A VH humanized amino acid sequence - SEQ ID NO: 42 
EVQLVESGGGLVQPGGSLKLSCAASGFTFSSFGMHWVRQAPEKGLEWVAYISSGSS
TIYYADTVKGRFIISRDNPKNTLFLQMTSLRSEDTAMYYCARKYEGYAMDYWGQGTL
VTVSS
IL13Rα2 143A VH murine nucleotide sequence - SEQ ID NO: 43 
GACGTGCAGCTGGTGGAAAGTGGAGGCGGTCTCGTCCAGCCAGGTGGTAGCAG
AAAGTTGAGCTGTGCCGCCAGTGGTTTTACATTCAGCTCCTTCGGAATGCACTGG
GTTCGTCAGGCACCCGAAAAGGGCCTGGAGTGGGTGGCCTATATATCCAGCGGC
AGTTCTACAATCTATTATGCCGACACAGTCAAAGGTAGGTTCATTATCTCCCGCGA
TAATCCCAAGAACACCCTGTTCTTACAGATGACCTCACTGCGGTCTGAGGATACA
GCTATGTACTACTGCGCTAGAAAATATGAGGGCTATGCTATGGACTACTGGGGAC
AGGGGACTTCCGTCACCGTGTCGTCG
IL13Rα2 143A VL murine nucleotide sequence - SEQ ID NO: 44 
GATATCAAGATGACCCAATCACCTTCTTCAATGTATGCAAGTCTTGGCGAAAGGGT
AACTATTACTTGCAAGGCCTCCCAAGACATCAACTCTTACCTCTCCTGGTTTCAGC
AGAAACCTGGCAAGAGTCCCAAAACGCTGATCTACCGGGCAAATCGCTTGGTTGA
CGGTGTGCCGAGCCGATTTTCAGGGAGCGGGTCTGGACAGGACTACAGCCTTAC
GATTTCTTCTCTGGAGTACGAGGATATGGGAATATACTCCTGTCTCCAATATGATG
AGTTTCCACTCACCTTCGGGGCGGGGACTAAGCTGGAACTAAAA
IL13Rα2 143A murine scFv amino acid sequence- SEQ ID NO: 45 
MALPVTGLLLSLGLLLHAARPTGDIKMTQSPSSMYASLGERVTITCKASQDINSYLSWF
QQKPGKSPKTLIYRANRLVDGVPSRFSGSGSGQDYSLTISSLEYEDMGIYSCLQYDEF
PLTFGAGTKLELKGGGGSGGGGSGGGGSGGGGSDVQLVESGGGLVQPGGSRKLS
CAASGFTFSSFGMHWVRQAPEKGLEWVAYISSGSSTIYYADTVKGRFIISRDNPKNTL
FLQMTSLRSEDTAMYYCARKYEGYAMDYWGQGTSVTVSS
IL13Rα2 143A murine CAR amino acid sequence - SEQ ID NO: 46 
DIKMTQSPSSMYASLGERVTITCKASQDINSYLSWFQQKPGKSPKTLIYRANRLVDGV
PSRFSGSGSGQDYSLTISSLEYEDMGIYSCLQYDEFPLTFGAGTKLELKGGGGSGGG
GSGGGGSGGGGSDVQLVESGGGLVQPGGSRKLSCAASGFTFSSFGMHWVRQAPE
KGLEWVAYISSGSSTIYYADTVKGRFIISRDNPKNTLFLQMTSLRSEDTAMYYCARKYE
GYAMDYWGQGTSVTVSSTTTPAPRPPTPAPTIASQPLSLRPEACRPAAGGAVHTRGL
DFACDIYIWAPLAGTCGVLLLSLVITLYCKRGRKKLLYIFKQPFMRPVQTTQEEDGCSC
RFPEEEEGGCELRVKFSRSADAPAYQQGQNQLYNELNLGRREEYDVLDKRRGRDPE
MGGKPQRRKNPQEGLYNELQKDKMAEAYSEIGMKGERRRGKGHDGLYQGLSTATK
DTYDALHMQALPPR
IL13Rα2 143A humanized scFv amino acid sequence- SEQ ID NO: 47 
MALPVTGLLLSLGLLLHAARPTGDIQMTQSPSSLYASVGDRVTITCKASQDINSYLSWY
QQKPGKAPKTLIYRANRLVDGVPSRFSGSGSGQDYTLTISSLQYEDMGIYSCLQYDEF
PLTFGAGTKVEIKGGGGSGGGGSGGGGSGGGGSEVQLVESGGGLVQPGGSLKLSC
AASGFTFSSFGMHWVRQAPEKGLEWVAYISSGSSTIYYADTVKGRFIISRDNPKNTLF
LQMTSLRSEDTAMYYCARKYEGYAMDYWGQGTLVTVSS
IL13Rα2 143A humanized CAR amino acid sequence- SEQ ID NO: 48

DIQMTQSPSSLYASVGDRVTITCKASQDINSYLSWYQQKPGKAPKTLIYRANRLVDGV
PSRFSGSGSGQDYTLTISSLQYEDMGIYSCLQYDEFPLTFGAGTKVEIKGGGGSGGG
GSGGGGSGGGGSEVQLVESGGGLVQPGGSLKLSCAASGFTFSSFGMHWVRQAPE
KGLEWVAYISSGSSTIYYADTVKGRFIISRDNPKNTLFLQMTSLRSEDTAMYYCARKYE
GYAMDYWGQGTLVTVSSTTTPAPRPPTPAPTIASQPLSLRPEACRPAAGGAVHTRGL
DFACDIYIWAPLAGTCGVLLLSLVITLYCKRGRKKLLYIFKQPFMRPVQTTQEEDGCSC
RFPEEEEGGCELRVKFSRSADAPAYQQGQNQLYNELNLGRREEYDVLDKRRGRDPE
MGGKPQRRKNPQEGLYNELQKDKMAEAYSEIGMKGERRRGKGHDGLYQGLSTATK
DTYDALHMQALPPR

### IL13Rα2_82B:

IL13Rα2_82B VL-CDR1- SEQ ID NO: 49: TASSSVSSSYLH
IL13Rα2_82B VL-CDR2- SEQ ID NO: 50: STSNLAS
IL13Rα2_82B VL-CDR3-SEQ ID NO: 51: HQYHRSPLT
IL13Rα2_82B VH-CDR1 - SEQ ID NO: 52: SYGVH
IL13Rα2_82B VH-CDR2- SEQ ID NO: 53: VIWSGGSTDYNAAFIS
IL13Rα2_82B VH-CDR3-SEQ ID NO: 54: DRYDAFDY
IL13Rα2 82B VL murine amino acid sequence - SEQ ID NO: 55 
QIVLTQSPAIMSASLGERVTMTCTASSSVSSSYLHWYQQKPGSSPKLWIYSTSNLAS
GVPARFSGSGSGTSYSLTISSMEAEDAATYYCHQYHRSPLTFGAGTKLELK
IL13Rα2 82B VH murine amino acid sequence - SEQ ID NO: 56 
QVQLKQSGPGLVQPSQSLSITCTVSGFSLTSYGVHWVRQSPGKGLEWLGVIWSGGS
TDYNAAFISRLSISKDNSKSQVFFKMNSLQANDTAIYYCARDRYDAFDYWGQGTTLTV
SS
IL13Rα2 82B VL humanized amino acid sequence - SEQ ID NO: 57 
EIVLTQSPSILSASVGEDVTITCTASSSVSSSYLHWYQQKPGSAPKLWIYSTSNLAS
GVPSRFSGSGSGTSFTLTISSLQAEDAATYYCHQYHRSPLTFGAGTKLEIK
IL13Rα2 82B VH humanized amino acid sequence - SEQ ID NO: 58 
QVQLQESGPGLVRPSQSLSLTCTVSGFSLTSYGVHWVRQSPGRGLEWLGVIWSGGS
TDYNAAFISRVTMSKDNSKNQVFFRMNSLQANDTAVYYCARDRYDAFDYWGQGTTV
TVSS
IL13Rα2 82B VH murine nucleotide sequence - SEQ ID NO: 59 
CAGGTTCAACTTAAACAGTCTGGACCCGGACTGGTTCAGCCAAGTCAGAGCCTAT
CCATCACCTGTACAGTAAGCGGTTTCTCCCTCACCTCCTATGGGGTGCATTGGGT
GCGACAGTCTCCTGGGAAGGGGCTGGAATGGTTGGGCGTCATCTGGAGCGGGG
GCAGCACCGATTACAATGCTGCCTTCATTTCAAGGCTCAGCATCAGTAAAGACAAT
AGCAAGTCCCAGGTCTTCTTCAAAATGAACAGTCTGCAGGCGAACGACACAGCCA
TATACTACTGTGCCCGTGACCGGTATGATGCGTTTGATTATTGGGGGCAAGGAAC
TACGCTGACCGTGAGCAGT
IL13Rα2 82B VL murine nucleotide sequence - SEQ ID NO: 60 
CAAATAGTGCTCACACAATCACCCGCAATTATGTCCGCATCTCTGGGAGAAAGAG
TGACAATGACGTGCACCGCATCATCTTCTGTCTCCAGTTCCTACCTCCACTGGTAT
 
CAGCAGAAGCCCGGCTCAAGCCCAAAGTTATGGATTTACTCTACTAGCAACTTAG
CCTCCGGCGTGCCTGCTCGCTTTTCCGGTTCGGGCTCTGGCACCTCATATTCGTT
GACTATCTCTTCAATGGAGGCTGAGGACGCTGCCACTTATTACTGCCATCAGTAC
CACAGGTCACCGCTGACTTTTGGCGCCGGGACAAAACTGGAGCTTAAG
IL13Rα2 82B murine scFv amino acid sequence- SEQ ID NO: 61 
MALPVTGLLLSLGLLLHAARPTGQIVLTQSPAIMSASLGERVTMTCTASSSVSSSYLH
WYQQKPGSSPKLWIYSTSNLASGVPARFSGSGSGTSYSLTISSMEAEDAATYYCHQY
HRSPLTFGAGTKLELKGGGGSGGGGSGGGGSGGGGSQVQLKQSGPGLVQPSQSL
SITCTVSGFSLTSYGVHWVRQSPGKGLEWLGVIWSGGSTDYNAAFISRLSISKDNSKS
QVFFKMNSLQANDTAIYYCARDRYDAFDYWGQGTTLTVSS
IL13Rα2 82B murine CAR amino acid sequence - SEQ ID NO: 62 
QIVLTQSPAIMSASLGERVTMTCTASSSVSSSYLHWYQQKPGSSPKLWIYSTSNLASG
VPARFSGSGSGTSYSLTISSMEAEDAATYYCHQYHRSPLTFGAGTKLELKGGGGSGG
GGSGGGGSGGGGSQVQLKQSGPGLVQPSQSLSITCTVSGFSLTSYGVHWVRQSPG
KGLEWLGVIWSGGSTDYNAAFISRLSISKDNSKSQVFFKMNSLQANDTAIYYCARDRY
DAFDYWGQGTTLTVSSTTTPAPRPPTPAPTIASQPLSLRPEACRPAAGGAVHTRGLD
FACDIYIWAPLAGTCGVLLLSLVITLYCKRGRKKLLYIFKQPFMRPVQTTQEEDGCSCR
FPEEEEGGCELRVKFSRSADAPAYQQGQNQLYNELNLGRREEYDVLDKRRGRDPE
MGGKPQRRKNPQEGLYNELQKDKMAEAYSEIGMKGERRRGKGHDGLYQGLSTATK
DTYDALHMQALPPR
IL13Rα2 82B humanized scFv amino acid sequence- SEQ ID NO: 63 
MALPVTGLLLSLGLLLHAARPTGEIVLTQSPSILSASVGEDVTITCTASSSVSSSYLHWY
QQKPGSAPKLWIYSTSNLASGVPSRFSGSGSGTSFTLTISSLQAEDAATYYCHQYHR
SPLTFGAGTKLEIKGGGGSGGGGSGGGGSGGGGSQVQLQESGPGLVRPSQSLSLT
CTVSGFSLTSYGVHWVRQSPGRGLEWLGVIWSGGSTDYNAAFISRVTMSKDNSKNQ
VFFRMNSLQANDTAVYYCARDRYDAFDYWGQGTTVTVSS
IL13Rα2 82B humanized CAR amino acid sequence- SEQ ID NO: 64

EIVLTQSPSILSASVGEDVTITCTASSSVSSSYLHWYQQKPGSAPKLWIYSTSNLASGV
PSRFSGSGSGTSFTLTISSLQAEDAATYYCHQYHRSPLTFGAGTKLEIKGGGGSGGG
GSGGGGSGGGGSQVQLQESGPGLVRPSQSLSLTCTVSGFSLTSYGVHWVRQSPGR
GLEWLGVIWSGGSTDYNAAFISRVTMSKDNSKNQVFFRMNSLQANDTAVYYCARDR
YDAFDYWGQGTTVTVSSTTTPAPRPPTPAPTIASQPLSLRPEACRPAAGGAVHTRGL
DFACDIYIWAPLAGTCGVLLLSLVITLYCKRGRKKLLYIFKQPFMRPVQTTQEEDGCSC

RFPEEEEGGCELRVKFSRSADAPAYQQGQNQLYNELNLGRREEYDVLDKRRGRDPE
MGGKPQRRKNPQEGLYNELQKDKMAEAYSEIGMKGERRRGKGHDGLYQGLSTATK
DTYDALHMQALPPR

### COMMON SEQUENCES:

Peptide linker - SEQ ID NO: 65: GGGGSGGGGSGGGGSGGGGS
Signal peptide - SEQ ID NO: 66: MALPVTGLLLSLGLLLHAARPTG
Hinge and Transmembrane domain derived from CD8-SEQ ID NO: 67: 
TTTPAPRPPTPAPTIASQPLSLRPEACRPAAGGAVHTRGLDFACDIYIWAPLAGTCGV
LLLSLVITLYC
Intracellular signaling domain derived from CD3ζ - SEQ ID NO: 68: 
RVKFSRSADAPAYQQGQNQLYNELNLGRREEYDVLDKRRGRDPEMGGKPQRRKNP
QEGLYNELQKDKMAEAYSEIGMKGERRRGKGHDGLYQGLSTATKDTYDALHMQALP
PR
Costimulatory signaling domain derived from CD137 - SEQ ID NO: 69: KRGRKKLLYIFKQPFMRPVQTTQEEDGCSCRFPEEEEGGCEL

### EXAMPLES

### Materials and Methods

### Generation of anti-IL13Ra2 antibodies and derived anti-IL13Ra2 single chain variable fragments (scFv)

The 6A, 46A, 82B and 143A murine anti-IL13Rα2 monoclonal antibodies (mAb) were generated at the Dr. Pablo Engel's lab (IDIBAPS, Universidad de Barcelona) using conventional mAb generation techniques. In brief, mAbs reactive with human IL13Rα2 were generated by fusion of NS-1 myeloma cells with splenocytes from a Balb/c mouse previously immunized three times with IL13Rα2-transfected 300.19 cells. Supernatants from hybridoma-containing wells were screened by flow cytometry for mAbs reactive with IL13Rα2-transfected 300.19 cells. Four reactive hybridomas were chosen for further characterization and were subcloned by limiting dilution (clones 6A, 46A, 82B and 143A). The four antibodies were IgG1κ isotype, as determined using a mouse mAb isotyping kit (Boehringer Mannheim). Antibody specificity was further validated using an IL13Rα2-transfected COS cell line and the cell line U251.

The sequence corresponding to the variable light (V_{L}) and variable heavy (V_{H}) regions of 6A and 46A antibodies was extracted from the corresponding hybridoma cells using Mouse Ig-Primer Set (Cat# 69831-3; Novagen). We used the Integrated antibody sequence & structure analysis (abYsis) web-based platform to analyze the generated hybridomas sequences. The sequence of V_{L} and V_{H} regions of 82B and 143A antibodies were obtained by Absolute Antibody (Oxford, UK).

We used the Integrated antibody sequence & structure analysis (abYsis) web-based platform to analyze the generated hybridoma sequences.

### Humanization of anti-IL13Rα2 scFv

Based on the murine sequences obtained from the hybridomas, a methodology was designed to generate humanized sequences. These humanized sequences were constructed by Complementarity Determining Regions (CDR) grafting, while retaining those murine framework residues that influence the antigen-binding activity and the structural configuration.

Briefly, the CDRs of both light and heavy chains were determined by several predictive algorithms like Kabat and Chothia numbering scheme, using Abysis and BLAST software. We also determined the most conserved positions of the sequences (>75% frequency) and identify the positions susceptible to post-translational modifications. These identified amino acids were maintained in the humanized sequence.

Next, a comparative search was made with the Position-Specific Iterated BLAST software to find the immunoglobulins of human origin with a closer identity to our sequence and will be used as a model to make the changes, but always respecting the essential positions identified above. Moreover, only synonymous amino acid changes were introduced. At this moment, a humanized sequence has been designed for the light and heavy chain variable regions of each murine scFv.

### Design of anti-IL13Rα2 and lentiviral production

Second-generation CARs were designed to contain the anti-IL13Rα2 scFvs (6A, 46A, 82B and 143A), the CD8 hinge and transmembrane domains, along with the 4-1 BB and CD3ζ endodomains. All constructs were synthesized by GenScript (Rijswijk, Netherlands) and cloned into a third-generation lentiviral vector pCCL (kindly provided by Dr. Luigi Naldini; San Raffaele Hospital, Milan). The expression of the CAR was controlled under the EF1α promoter.

The anti-IL13Rα2 CARs lentiviral vectors were produced by transfecting HEK293FT cells using PEI (Cat# 23966-100; Polysciences). The lentivirus-containing supernatant were collected after 48 hours and 72 hours and concentrated using Lenti-X Concentrator (Cat# 631232; Takara Bio). Lentiviral vectors were tittered based on superficial CAR expression using wild-type Jurkat cells, using the limiting dilution method.

### CAR-T cell generation

Human primary T lymphocytes were isolated from buffy coats of healthy volunteer donors (Banc de Sang i Teixits, Barcelona, Spain) using density gradient centrifugation with Lymphoprep (Cat# 07801; StemCell). CD3+ T lymphocytes were separated using RosetteSep for Human T cells (Cat# 15061; StemCell) and stimulated with CD3/CD28 Human T-Activator Dynabeads (Cat# 11131D; Gibco BRL Life Technologies). T lymphocytes were cultured in complete RPMI 1640 media (Cat# 21875034; Gibco BRL Life Technologies) containing 10% heat-inactivated fetal bovine serum (FBS) (Cat# F9665-500ML, Sigma), 1% penicillin-streptomycin (Cat# 15140-122; Gibco BRL Life Technologies), 1% GlutaMax (Cat# 35050-061; Thermo Fisher Scientific), human IL-2 (50 IU/mL; Cat# 130-097-743; Miltenyi Biotec). Stimulated T lymphocytes were then transduced with the corresponding lentiviral vector encoding the scFv-based constructs targeting IL13Rα2 on day 1 of expansion, as specified. On day 6 of expansion, activation beads were magnetically removed from all groups.

### Cell lines maintenance

THP1 cell line, expressing GFP and luciferase (LUC), was generously donated by Dr Nela Klein and U87MG cell line was kindly provided by Dr Carlos Barcia. The THP1 cell line was maintained in RPMI 1640 media (Gibco BRL Life Technologies) supplemented with 10% heat-inactivated FBS (Cat# F9665-500ML, Sigma) and 1% penicillin-streptomycin (Gibco BRL Life Technologies), and 1% GlutaMax (Cat# 35050-061; Thermo Fisher Scientific). The HEK293FT (Cat# R70007, Invitrogen, Waltham, MA, USA) and U87MG cell lines were maintained in DMEM media (Cat# 10313021; Thermo Fisher Scientific) containing 10% heat-inactivated FBS (Cat# F9665-500ML, Sigma), 1% penicillin-streptomycin (Gibco BRL Life Technologies), and 1% GlutaMax (Cat# 35050-061; Thermo Fisher Scientific). All cell lines were expanded according to ATCC recommendations. Cells were maintained in a humidified atmosphere containing 5% carbon dioxide (CO₂) at 37°C. All cell lines were routinely tested for Mycoplasma.

GFP and LUC-expressing U87MG (U87MG GFP-LUC) cell line were generated by transducing the parental cell line with a lentiviral vector (kindly provided by Dr Nela Klein) encoding the Click Beetle Green (CBG) luciferase and GFP, connected by a T2A self-cleavage peptide, under the EF1α promoter. Transduction efficiency was confirmed by flow cytometry and GFP positive cells were sorted to establish the U87MG GFP-LUC cell line.

### Flow cytometry and antibodies

To assess CAR expression on the cell surface, CAR T cells were incubated with biotin-SP AffiniPure Goat Anti-Mouse IgG (Cat# 115-065-072; Jackson ImmunoResearch) for 30 minutes at 4°C. Subsequently, cells were stained with PE-conjugated streptavidin (Cat# 12-4317-87; eBioscience) for 20 minutes at room temperature. For the T cell subsets panel the following fluorochrome-conjugated mAbs were used: CCR7-PerCP Cy5.5 (BD Biosciences; 561144), CD62L-FITC (Cat# 555543; BD Biosciences), CD8-APC H7 (Cat# 560179; BD Biosciences), CD45RA-PeCy7 (Cat# 560675; BD Biosciences). In the case of the T cell exhaustion panel, we employed: PD1-APC (Cat# 558694; BD Biosciences), CTLA4-PE (Cat# 557301; BD Biosciences), TIM3-FITC (Cat# 11-3109-42; eBioscience), LAG3-PeCy7 (Cat# 25-2239-42; eBioscience), CD8-APC H7 (Cat# 560179; BD Biosciences), CD28-PerCP Cy5.5 (Cat# 337181; BD Biosciences). In the *in vivo* model, T cell persistence was examined staining for human CD45+ (#Cat 555485; BD Pharmingen), and CAR was detected with biotin-SP AffiniPure Goat Anti-Mouse IgG (Cat# 115-065-072; Jackson ImmunoResearch) and BV421-conjugated streptavidin (Cat# 563259; BD Horizon). All flow cytometry data were obtained in Attune NxT (ThermoFisher Scientific) and analyzed with FlowJo software.

### In vitro coculture experiments

For luciferase-based assay coculture experiments, freshly collected CAR T cells from a day 8 expansion were cocultured with a fixed number (1 × 10⁴) of tumor cells (THP1 or U87MG) expressing GFP-LUC at effector:target (E:T) ratios of 8:1, 4:1, 2:1, 1:1, 0.5:1 and 0.25:1. Cells were seeded in duplicate for each ratio in a white opaque 96-well plate. After 24 hours, cytotoxicity was measured (Synergy HT, Biotek) with the addition of 3 µL with D-Luciferin (20 mg/mL diluted in PBS; Cat# 122799; Perkin Elmer). Then, cells were harvested in duplicate, and supernatants collected. For the cytokine production assay, coculture supernatants were analyzed for cytokine production (IFNγ, TNFα, and Granzyme B) by ELISA, following the manufacturer's instructions (R&D systems duo set, Human IFNγ DTY285B-05, Human Granzyme B DY2906-05, TNFα DY210-05). All experiments were run, at least, in triplicate.

### Glioblastoma (GBM) mouse xenograft model

For the *in vivo* experiments, immune-compromised NOD-SCID-Gamma (NSG) male mice (aged 8-12 weeks) were engrafted with 1 × 10⁶ U87MG GFP-LUC cells by subcutaneous injection in the back. After three weeks, a single dose of 1 or 5 × 10⁶ CAR+ (6A or 46A) T cells was injected intravenously into the mice. In other experiment, NSG mice were injected subcutaneously with 1×10⁶ U87MG GFP-LUC cells, and 24 hours later animals were treated with a single injection of 3×10⁶ CAR+ (6A or 46A) T cells. T cells showed a CAR expression ranging between 15-35%. Tumor growth was monitored twice a week via caliper (tumor volume).

To analyze T cell persistence, peripheral blood samples were obtained by submaxillary bleeding at the indicated timepoints. Spleen and tumor were also collected at the end point of the experiment in each animal to assess T cell infiltration. Samples were firstly lysed (#Cat 10-548E; Lonza) and blocked with FcR Blocking reagent (#Cat 130-092-575; Miltenyi). Then, T cell persistence was examined staining for human CD45 and CAR. All procedures were conducted in compliance with the Animal Ethics Committee of the Universitat de Barcelona (27/21 P2).

### Statistical analysis

Graphs and statistical analysis were performed using GraphPad Prism Software version 8.3. All data are presented as mean ± standard error of mean (SEM), except when indicated. One-way ANOVA test was used multiple comparisons in a single condition. Statistical analysis of tumor flux was performed using a two-way ANOVA test. Kaplan-Meier survival data were analyzed using a log rank (Mantel-Cox) test. In case of using another statistical test, it is specified in the figure caption. P-values are represented as follows: not significant (ns), * (p < 0.05), ** (p < 0.01), *** (p < 0.001) or **** (p < 0.0001).

### Results

### Design of the scFvs targeting IL13Rα2.

To establish the single chain variable fragments (scFvs) to be used in the design of the anti-IL13Rα2 chimeric antigen receptor (CAR) constructs, four different anti-IL13Rα2 hybridomas were sequenced. These four hybridomas, whose specificity was previously tested, are known as IL13Rα2_6A, IL13Rα2_46A, IL13Rα2_82B, and IL13Rα2_143A. Their corresponding cDNA was synthesized from total RNA of each hybridoma cells. Genes encoding light chain (V_{L}) and heavy chain (V_{H}) variable regions of each monoclonal antibody against IL13Rα2 were amplified by PCR and sequenced (Fig. 1). V_{L} fragments size were 321 bp for IL13Rα2_6A and IL13Rα2_143A, and 324 bp for IL13Rα2_46A and IL13Rα2_82B. V_{H} fragments size were 354 bp for IL13Rα2_6A and IL13Rα2_143A, and 348 bp for IL13Rα2_46A and IL13Rα2_82B. Then, the amino acid sequences from the complementarity-determining regions (CDRs) 1, 2 and 3 of both V_{L} and V_{H} were identified according to the method provided by the Integrated antibody sequence & structure analysis (abYsis) web-based platform, using Kabat scheme.

The predicted CDR1, CDR2 and CDR3 of V_{L} domain were located at positions 24-34, 50-56, and 89-97 in the IL13Rα2_6A protein sequence; 24-35, 51-57, and 90-98 in the IL13Rα2_46A protein sequence; 24-35, 51-57, and 90-98 in the IL13Rα2_82B protein sequence; and 24-34, 50-56, and 89-97 in IL13Rα2_143A in the protein sequence, respectively (Fig. 2A). The prediction of CDR1, CDR2 and CDR3 of V_{H} domain was specified at positions 31-35, 50-66, and 99-107 in the IL13Rα2_6A protein sequence; 31-35, 50-65, and 98-105 in the IL13Rα2_46A protein sequence; 31-35, 50-65, and 98-105 in the IL13Rα2_82B protein sequence; and 31-35, 50-66, and 99-107 in IL13Rα2_143A in the protein sequence, respectively (Fig. 2B).

To produce full-length scFvs derived from the anti-IL13Rα2 antibodies, a 20 amino acid linker encoding (Gly₄Ser)₄ was introduced between the V_{L} and the V_{H} domains (Fig. 2C).

### Development of anti-IL13Rα2 CAR (CARIL13Rα2) T cells.

To generate anti-IL13Rα2 CAR (CARIL13Rα2) T cells, the four scFvs were cloned into a second-generation CAR backbone. These anti-IL13Rα2 CARs contain a CD8 hinge and transmembrane domain, along with the 4-1BB and CD3ζ intracellular signaling domains. To compare the four CAR T cell products (CARIL13Rα2_6A, CARIL13Rα2_46A, CARIL13Rα2_82B and CARIL13Rα2_143A), we generated CARIL13Rα2 T cells by transducing T cells with lentiviral vectors including the transgene illustrated in Fig. 3A. Primary CD4+ and CD8+ T lymphocytes were isolated, stimulated 24 hours prior to lentiviral transduction and then expanded for 10 days in culture. Transduction of T lymphocytes with lentiviral vectors resulted in an efficient expression of the four CARIL13Rα2 constructs, detected by flow cytometry on day 8 of T cell *ex vivo* expansion (Fig. 3B).

CAR T cells proliferation in terms of population doublings during the expansion showed that each CARIL13Rα2 T cells had an indistinguishable proliferation kinetics from the untransduced (UT) control T cells. On the contrary, CARIL13Rα2_82B expanded significantly less than CARIL13Rα2_143A T cells at day 10 (Fig. 4A). Regarding CAR expression on surface, any difference was observed in CAR expression levels at day 8 between CARIL13Rα2_6A, CARIL13Rα2_46A and CARIL13Rα2_143A T cells, with a range between 20-40%. Surprisingly, the CARIL13Rα2_82B expression was higher in comparison to the rest of the constructs (Fig. 4B). The CD4/CD8 ratio was similar at day 8 between UT, CARIL13Rα2_6A, CARIL13Rα2_46A, CARIL13Rα2_82B and CARIL13Rα2_143A T cells (Fig. 4C).

### Anti-IL13Rα2 CAR T cells get activated and kill specifically IL13Ra2+ derived glioblastoma cells in vitro.

To assess the antitumor efficacy and specificity of the anti-IL13Rα2 CAR T cells, these T cells were cocultured with the IL13Rα1+/IL13Rα2+ glioblastoma (GBM) derived U87MG, and IL13Rα1+/IL13Rα2- monocytic leukemia derived THP1 cell lines^{1,2}. Both cell lines were labeled with Click Beetle Green luciferase and GFP. The four CARIL13Rα2 T cells exhibited potent cytotoxicity against the GBM derived U87MG cell line after 24 hours, as determined by a luciferase-based assay (Fig. 5A). However, this antitumor effect was not seen against THP1 cell line, highlighting the specificity against IL13Rα2 (Fig. 5B).

To evaluate cytokine production, CARIL13Rα2 T cells and U87MG tumor cells were cocultured for 24 hours at a 2:1, and 1:1 E:T ratio (Fig. 6). CAR-mediated activation was confirmed by the production of IFNγ (Fig. 6A), TNFα (Fig. 6B), and granzyme B (Fig. 6C). In the case of UT control T cells, the levels were lower than the detection limit for each of the three proinflammatory cytokines measured. Although the high variability between different donors, CARIL13Rα2_6A, CARIL13Rα2_46A, CARIL13Rα2_82B and CARIL13Rα2_143A showed an important increase in IFNγ and granzyme B levels at 2:1 and 1:1 E:T ratio. While for TNFα and granzyme B levels any significant difference was observed between conditions at any E:T ratio; CARIL13Rα2_6A and CARIL13Rα2_82B showed a slightly higher IFNγ production in comparison to CARIL13Rα2_143A at 2:1 E:T ratio. This difference was maintained between CARIL13Rα2_6A and CARIL13Rα2_143A at 1:1 E:T ratio. As expected, CARIL13Rα2_6A, CARIL13Rα2_46A, CARIL13Rα2_82B and CARIL13Rα2 _143A did not show any increase in IFNγ, TNFα and granzyme B levels in the cocultures with THP1 cells (data not shown), indicating the absence of reactivity against IL13Rα1 and the ability to recognize only the target antigen.

### Immunophenotypic characterization of CARIL13Ra2 T cells.

In order to characterize the subpopulations of CARIL13Rα2 T cells present in the culture after 10 days of expansion, CD45RA, CD62L, and CCR7 markers were analyzed by flow cytometry. Naive (CD62L+ CD45RA+), central memory (CD62L+ CD45RA-), effector memory (CD62L- CD45RA-) and effector (CD62L- CD45RA+) T cell subpopulations were reported (Fig. 7). In all cases, most T cells belonged to the central memory and effector memory T subsets, as expected based on the group's experience with other CAR T cell therapies. Any statistical difference was observed in total (Fig. 7A) or CD4+ (Fig. 7B) T cells when comparing different CARIL13Rα2 constructs. In CD8+ (Fig. 7C) T cells, the levels of naive T cells were slightly higher in UT control T cells than in CARIL13Rα2_6A T cells.

The surface expression of CTLA4, LAG3, PD1 and TIM3 was determined by flow cytometry to evaluate the presence of T cell exhaustion markers in the total T cell population (Fig. 8A) and in CD4+ (Fig. 8B) or CD8+ (Fig. 8C) T cells. Although the different CARIL13Rα2 showed a higher expression of CLTA4, LAG3, PD1 and TIM3; no statistical difference was observed, probably due to the high heterogeneity between donors. The same was observed for CD28 antigen expression (Fig. 9).

### Humanized CARIL13Rα2 T cells.

Based on the murine sequences obtained from the IL13Rα2 hybridomas (IL13Rα2_6A, IL13Rα2_46A, IL13Rα2_82B and IL13Rα2_143A) we humanized sequences by CDR grafting, while retaining those murine framework residues that influence the antigen-binding activity and the structural configuration (Fig. 10).

### CARIL13Rα2_6A and 46A T cells and GBM progression in a mouse xenograft model after a single intravenous injection of 1×10⁶ CAR+ T cells.

To assess the *in vivo* antitumor activity of CARIL13Rα2_6A and CARIL13Rα2_46A T cells, NOD-SCID-Gamma (NSG) animals were engrafted with U87MG GFP-LUC cells (Fig. 11A). Three weeks after tumor engraftment, animals were treated with a single intravenous injection of 1×10⁶ of UT or 1×10⁶ CARIL13Rα2_6A+ or CARIL13Rα2_46A+ T cells. Tumor growth was monitored twice a week via caliper (tumor volume). The CARIL13Rα2_6A and CARIL13Rα2_46A T cells were able to control tumor progression (Fig. 11B) until day 40 post tumor challenge, resulting in a reduction in tumor volume compared to the UT control group. However, the CARIL13Rα2_46A T cells showed a similar tumor volume than UT control cells in day 42. The antitumor activity of both CAR T cells led to an improvement in the survival of mice treated with these cells compared to the control groups (Fig. 11C).

T cell persistence in the peripheral blood of treated mice was measured at different timepoints after CAR T cell treatment (Fig. 12), reflecting the proliferation of both CAR T cells between day 10 and 20 after the treatment injection. Moreover, spleen and tumor were collected at the end point of the experiment to assess T cell infiltration (Fig. 13). The CARIL13Rα2_6A and CARIL13Rα2_46A T cells demonstrated migration ability, suggesting that T cell trafficking toward until the tumor took place *in vivo.*

### CARIL13Rα2_6A and 46A T cells control GBM progression in a mouse xenograft model after a single intravenous injection of 5×10⁶ CAR+ T cells.

To enhance the *in vivo* antitumor activity of CARIL13Rα2_6A and CARIL13Rα2_46A CAR T cells, the NSG animals were treated with a single intravenous injection of 5×10⁶ CARIL13Rα2_6A+ or CARIL13Rα2_46A+ T cells, 24 days after U87MG GFP-LUC cell engraftment (Fig. 14A). The CARIL13Rα2_6A T cells were able to control tumor progression (Fig. 14B), resulting in a significant reduction in tumor burden compared to the control UT group. Moreover, the CARIL13Rα2_46A CAR T cells also exhibited a potent *in vivo* antitumor activity. The robust antitumor activity of CARIL13Rα2_6A CAR T cells led to a significant improvement in the survival of mice treated with these cells compared to the control group (Fig. 14C). However, the CARIL13Rα2_46A CAR T cells showed a limited capacity to eradicate the tumor in this *in vivo* model.

As expected, T cell persistence in the peripheral blood of treated mice was also evidenced for both CARIL13Rα2_6A and CARIL13Rα2_46A T cells, but with great heterogeneity between individuals (Fig. 15). In the same manner, T cell infiltration was evaluated in spleen and tumor at the end point of the experiment. The CARIL13Rα2_6A and CARIL13Rα2_46A T cells demonstrated a higher infiltration in comparison to the UT control group (Fig. 16).

### CARIL13Rα2_6A and 46A T cells and GBM progression in a mouse xenograft model with a co-injection of tumor cells and a single intravenous injection of 3×10⁶ CAR+ T cells

With the aim of assessing whether the CARIL13Rα2_6A and CARIL13Rα2_46A T cells can avoid tumor development before the U87MG engraftment, NSG mice were injected subcutaneously with 1×10⁶ U87MG GFP-LUC cells, and 24 hours later animals were treated with a single injection of 3×10⁶ CARIL13Rα2_6A+ or CARIL13Rα2_46A+ T cells (Fig. 17A). While CARIL13Rα2_46A T cells were able to control tumor progression, no remarkable effect was seen for CARIL13Rα2_6A T cells in comparison to the UT control cells (Fig. 17B). This difference in the antitumor effect led to an improvement in the survival of mice treated with CARIL13Rα2_46A+ T cells (Fig.17C).

As previously described, T cell persistence in the peripheral blood of treated mice was also measured at different timepoints after CAR T cell treatment. Although CARIL13Rα2_6A+ and CARIL13Rα2_46A+ T cells persisted in an expected manner, UT control cells showed a high proliferation in some individuals, probably indicating an unspecific and allogenic reaction (Fig. 18). The same pattern was observed when T cell infiltration was evaluated in spleen and tumor at the end point of the experiment (Fig. 19).

### Bibliography

1. Kong, S. et al. Suppression of Human Glioma Xenografts with Second-Generation IL13R-Specific Chimeric Antigen Receptor-Modified T Cells. Clinical Cancer Research 18, 5949-5960 (2012).
2. Muhammad, N. et al. A novel TanCAR targeting IL13Rα2 and EphA2 for enhanced glioblastoma therapy. Mol Ther Oncolytics 24, 729-741 (2022).

## Claims

1. An **IL13Rα2 targeting moiety** comprising:
- a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 1, LCDR2 consists of SEQ ID NO: 2, LCDR3 consists of SEQ ID NO: 3, HCDR1 consists of SEQ ID NO: 4, HCDR2 consists of SEQ ID NO: 5, and HCDR3 consists of SEQ ID NO: 6, or
- a VL domain and VH domain, wherein said VL domain comprises LCDR1, LCDR2 and LCDR3 polypeptides and said VH domain comprises HCDR1, HCDR2 and HCDR3 polypeptides, and LCDR1 consists of SEQ ID NO: 17, LCDR2 consists of SEQ ID NO: 18, LCDR3 consists of SEQ ID NO: 19, HCDR1 consists of SEQ ID NO: 20, HCDR2 consists of SEQ ID NO: 21, and HCDR3 consists of SEQ ID NO: 22.

2. The IL13Rα2 targeting moiety according to claim 1, wherein the IL13Rα2 targeting-moiety is an scFv comprising a VL and VH domains, and wherein:
- the VL domain comprises a sequence with at least 80% sequence identity to SEQ ID NO: 7, and wherein said VL domain comprises CDR polypeptides of LCDR1 according to SEQ ID NO: 1, LCDR2 according to SEQ ID NO: 2, and LCDR3 according to SEQ ID NO: 3, and said VH domain comprises a sequence with at least 80% sequence identity to SEQ ID NO: 8, and wherein said VH domain comprises CDR polypeptides of HCDR1 according to SEQ ID NO: 4, HCDR2 according to SEQ ID NO: 5, and HCDR3 according to SEQ ID NO: 6, or
- the VL domain comprises a sequence with at least 80% sequence identity to SEQ ID NO: 23, and wherein said VL domain comprises CDR polypeptides of LCDR1 according to SEQ ID NO: 17, LCDR2 according to SEQ ID NO: 18, and LCDR3 according to SEQ ID NO: 19, and said VH domain comprises a sequence with at least 80% sequence identity to SEQ ID NO: 24, and wherein said VH domain comprises CDR polypeptides of HCDR1 according to SEQ ID NO: 20, HCDR2 according to SEQ ID NO: 21, and HCDR3 according to SEQ ID NO: 22.

3. The IL13Rα2 targeting moiety according to any one of claims 1 or 2, wherein the IL13Rα2 targeting moiety is a scFv comprising a VL domain and VH domain, wherein:
- the VL domain consists of SEQ ID NO: 7 and the VH domain consists of SEQ ID NO: 8,
- the VL domain consists of SEQ ID NO: 9 and the VH domain consists of SEQ ID NO: 10,
- the VL domain consists of SEQ ID NO: 23 and the VH domain consists of SEQ ID NO: 24, or
- the VL domain consists of SEQ ID NO: 25 and the VH domain consists of SEQ ID NO: 26.

4. The IL13Rα2 targeting moiety according to any one of claims 1 to 3, wherein the IL13Rα2 targeting-moiety is a scFv consisting of SEQ ID NO: 13, 15, 29 or 31.

5. A chimeric antigen receptor (CAR) comprising:
a) an extracellular domain comprising a IL13Rα2 targeting moiety as defined in any one of claims 1 to 4;
b) a transmembrane domain; and
c) an intracellular signaling domain.

6. The CAR according to claim 5, wherein the transmembrane domain comprises the transmembrane domain of CD8.

7. The CAR according to any one of claims 5 or 6, wherein the intracellular signaling domain comprises the intracellular domain of CD3ζ.

8. The CAR according to any one of claims 5 to 7, wherein the CAR further comprises a costimulatory signaling domain, preferably the costimulatory signaling domain comprises the intracellular domain of CD137.

9. The CAR according to any one of claims 5 to 8, consisting of SEQ ID NO: 14, 16, 30 or 32.

10. A nucleic acid encoding the CAR according to any one of claims 5 to 9.

11. A cell comprising the nucleic acid according to claim 10 and/or the CAR according to any one of claims 5 to 9.

12. A pharmaceutical composition comprising a plurality of cells according to claim 11 and a pharmaceutically acceptable carrier or diluent.

13. The IL13Rα2 targeting moiety according to any one of claims 1 to 4, the CAR according to any one of claims 5 to 9, the nucleic acid according to claim 10, the cell according to claim 11, or the pharmaceutical composition according to claim 12, for use in therapy or as a medicament.

14. The IL13Rα2 targeting moiety according to any one of claims 1 to 4, the CAR according to any one of claims 5 to 9, the nucleic acid according to claim 10, the cell according to claim 11 or the pharmaceutical composition according to claim 12, for use in a method of treating or preventing a IL13Rα2-positive cancer, wherein the method comprises administering the IL13Rα2 targeting moiety, the CAR, the nucleic acid, the cell or the composition to a patient in need thereof.

15. The IL13Rα2 targeting moiety, the nucleic acid, the cell, or the pharmaceutical composition for use according to claim 14, wherein the IL13Rα2-positive cancer is glioblastoma or diffuse intrinsic pontine glioma.
